# EUROPEAN PATENT SPECIFICATION

(11) **EP 4 259 146 B1**
(45) Date of publication and mention of the grant of the patent: **02.12.2026**
(21) Application number: 21843814.1
(22) Date of filing: 06.12.2021
(51) Int. Cl.: A61K 31/506, A61K 31/517, A61K 31/5377, A61K 39/395, A61P 35/00, C07K 16/22

(54) **COMPOSITIONS COMPRISING A KRASG12C INHIBITOR AND A EGFR-INHIBITOR FOR TREATING SOLID TUMORS**
ZUSAMMENSETZUNGEN MIT EINEM KRASG12C-HEMMER UND EINEM EGFR-HEMMER ZUR BEHANDLUNG VON SOLIDEN TUMOREN
COMPOSITIONS COMPRENANT UN INHIBITEUR DE KRASG12C ET UN INHIBITEUR D'EGFR POUR TRAITER DES TUMEURS SOLIDES

(30) Priority: 08.12.2020 US 202063122702 P
(43) Date of publication of application: 18.10.2023
(73) Proprietor: Genentech, Inc., South San Francisco, CA 94080-4990 (US)
(72) Inventor: EVANGELISTA, Marie, San Francisco, California 94080-4990 (US); MERCHANT, Mark Andrew, San Francisco, California 94080-4990 (US); SCHUTZMAN, Jennifer Lee, San Francisco, California 94080-4990 (US); LIN, Ting-Kun Mark, San Francisco, California 94080-4990 (US); JOO, Stephanie Royer, San Francisco, California 94080-4990 (US); MANDLEKAR, Sandhya Vinayak, San Francisco, California 94080-4990 (US)
(74) Representative: Jochnowitz, Evan
(86) International application number: PCT/US2021/061980
(87) International publication number: WO 2022/125427

(56) References cited:
- WO-A1-2020/055756
- WO-A2-2020/097537
- WO-A2-2020/106647

## Description

### FIELD OF INVENTION

This application claims the benefit of U.S. Provisional Patent Application No. 63/122,702, filed 8 December 2020.

### FIELD OF INVENTION

Provided herein are combination therapies comprising a KRas^{G12C} inhibitor (Compound 1) and an EGFR-inhibitor and methods of using such combination therapies.

### BACKGROUND

The Kirsten rat sarcoma viral oncogene homolog (KRAS) is a central component of the RAS/MAPK signal transduction pathway, an intracellular network of proteins that transmit extracellular growth factor signals to regulate cell proliferation, differentiation, and survival. Mutations in KRAS can result in alterations at several amino acids, including glycine 12 (G12), glycine 13, and glutamine 61, commonly found in solid tumors and associated with tumorigenesis and aggressive tumor growth (Der et al. Proc Natl Acad Sci U S A 1982;79:3637-40; Parada et al. Nature 1982;297:474-8; Santos et al. Nature 1982;298:343-7; Taparowsky et al. Nature 1982;300:762-5; Capon et al. Nature 1983;304:507-13). Oncogenic KRAS mutations that result in the change from G12 to cysteine (G12C) are prevalent in non-small cell lung cancer (NSCLC) (~12%), colorectal cancer (CRC) (~4%), and other tumor types (≤ 4%) (Bailey et al. Nature 2016;531:47-52; Campbell et al. Nat Genet 2016;48:607-16; Giannakis et al. Cell Reports 2016;15:857-65; Hartmaier et al. Genome Med 2017;9(16); Jordan et al. Cancer Discov 2017;7:596-609).

Advanced stage tumors harboring the KRas^{G12C} mutation (hereafter referred to as KRas^{G12C}-positive tumors), including for example, lung cancer (e.g. NSCLC), CRC, and pancreatic cancer are incurable and carry a poor prognosis (Roman et al. Mol Cancer 2018;17:33; Wan et al. World J Gastroenterol 2019;25:808-23). In addition, patients with advanced stage KRas^{G12C}-positive cancers may derive limited benefit from select chemotherapies and targeted therapies, thus, restricting effective available treatment options (Roman et al. 2018).

WO 2020/106647 and WO 2020/055756 disclose the use of a combination of a KRAS^{G12C} inhibitor with an EGFR inhibitor in the treatment of lung, pancreatic, or colorectal cancer comprising a KRAS^{G12C} mutation.

Thus, there is a need for effective therapies and combination therapies for treating cancers such as lung cancer, colorectal cancer, and pancreatic cancer harboring KRas^{G12C} mutations.

### SUMMARY

Provided herein are solutions to these and other problems in the art. Note that the references to the methods of treatment by therapy of this description are to be interpreted as references to compounds, pharmaceutical compositions and medicaments of the present invention for use in those methods.

In one aspect provided herein is a combination therapy comprising Compound 1 or a pharmaceutically acceptable salt thereof as described herein; and an EGFR-inhibitor. The EGFR-inhibitor is erlotinib, gefitinib, osimertinib, dacomitinib, or afatinib or an anti-EGFR antibody. In one embodiment, the EGFR-inhibitor is erlotinib or cetuximab.

In another aspect provided herein is a combination therapy comprising Compound 1 or a pharmaceutically acceptable salt thereof as described herein administered QD on days 1-21 of a first 21-day cycle and erlotinib administered QD on days 1-21 of the first 21-day cycle.

In another aspect provided herein is a combination therapy comprising Compound 1 or a pharmaceutically acceptable salt thereof as described herein administered QD on days 1-21 of a first 21-day cycle and cetuximab administered Q1W starting on day 1 the first 21-day cycle.

In another aspect provided herein is a method of treating lung cancer mediated by a KRas^{G12C} mutation in a patient having such a lung cancer, the method comprising administering an effective amount of a combination therapy comprising Compound 1 or a pharmaceutically acceptable salt thereof as described herein administered QD on days 1-21 of a first 21-day cycle and an EGFR-inhibitor selected from the group consisting of erlotinib, gefitinib, osimertinib, dacomitinib, and afatinib. In one embodiment, the lung cancer is NSCLC.

In another aspect provided herein is a method of treating colorectal cancer (CRC) mediated by a KRas^{G12C} mutation in a patient having CRC, the method comprising administering an effective amount of a combination therapy comprising: Compound 1 or a pharmaceutically acceptable salt thereof as described herein administered QD on days 1-21 of a first 21-day cycle and an anti-EGFR antibody comprising panitumumab or cetuximab.

### BRIEF DESCRIPTION OF THE FIGURES

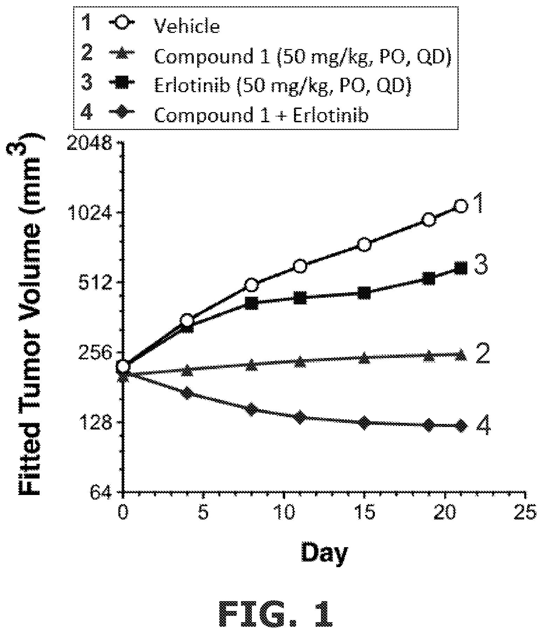

FIG. 1 illustrates the effect of Compound 1 and Erlotinib Dosed Alone or in Combination in NCI-H2122 NSCLC Tumor Xenografts in Nude Mice. Vehicles = 0.5% (w/v) methylcellulose; 0.5% (w/v) methylcellulose, 0.2% Tween 80^{™}. Fitted group tumor volumes after oral administration of Compound 1 or erlotinib dosed QD alone or in combination for 21 days are depicted. Dose levels are expressed as free-base equivalents.

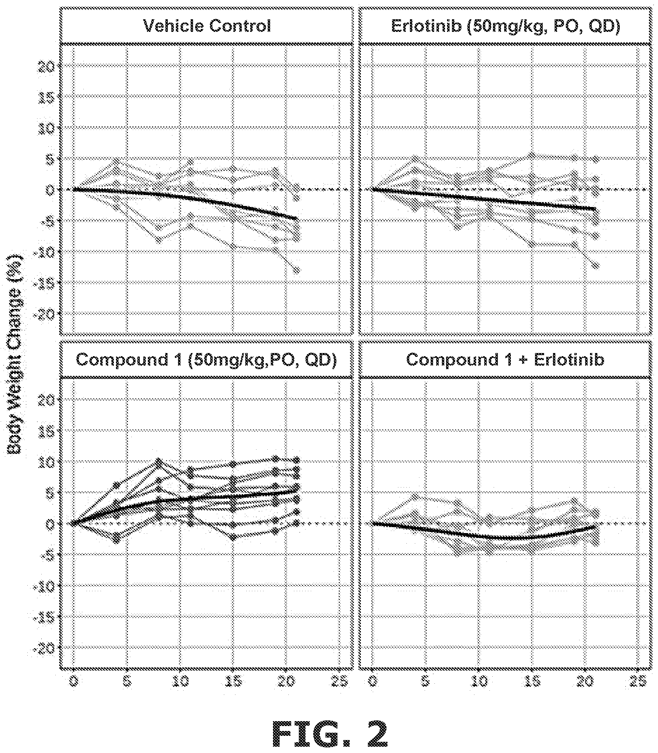

FIG. 2 illustrates Individual Body Weights Following Treatment With Compound 1 and Erlotinib Dosed Alone or in Combination in NCI-H2122 NSCLC Tumor Xenografts in Nude Mice. QD = once daily (21 times). Vehicles = 0.5% *(w*/*v)* methylcellulose (150 µL), 0.5% (w/v) methylcellulose/0.2% Tween 80^{™} (100 µL)

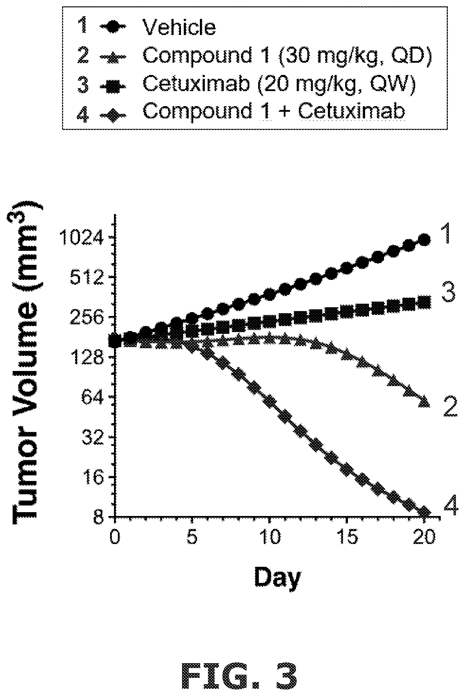

FIG. 3 illustrates the effect of Compound 1 and Cetuximab Dosed Alone or in Combination in CR6256 Colorectal Patient-Derived Xenograft Model in Nude Mice. Vehicles = 0.5% (w/v) methylcellulose; 0.5% (w/v) methylcellulose, 0.2% Tween 80^{™}. Fitted group tumor volumes after oral administration of Compound 1 dosed PO, QD or Cetuximab dosed IP, BIW alone or in combination for 21 days are depicted. Dose levels are expressed as free-base equivalents.

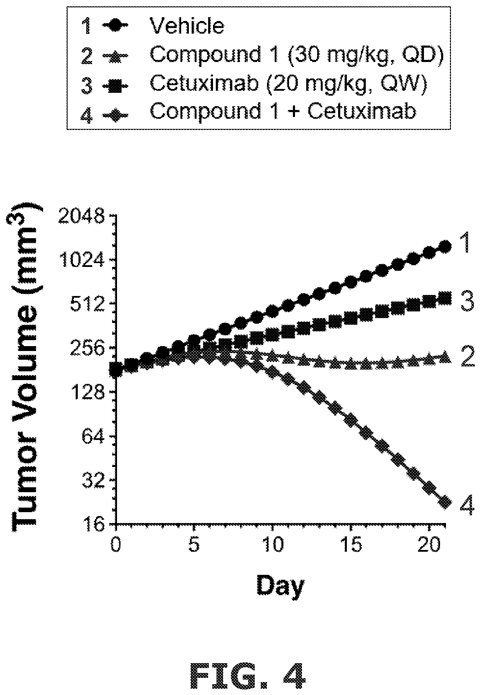

FIG. 4 illustrates the effect of Compound 1 and Cetuximab Dosed Alone or in Combination in CR5048 Colorectal Patient-Derived Xenograft Model in Nude Mice. Vehicles = 0.5% (w/v) methylcellulose; 0.5% (w/v) methylcellulose, 0.2% Tween 80^{™}. Fitted group tumor volumes after oral administration of Compound 1 dosed PO, QD or Cetuximab dosed IP, BIW alone or in combination for 21 days are depicted. Dose levels are expressed as free-base equivalents.

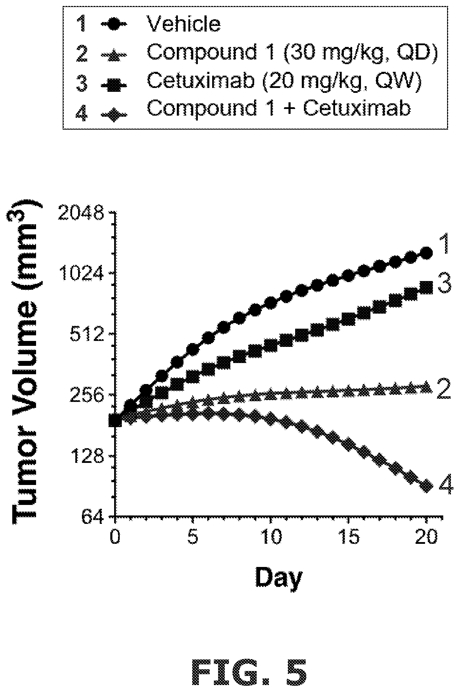

FIG. 5 illustrates the effect of Compound 1 and Cetuximab Dosed Alone or in Combination in CR6243 Colorectal Patient-Derived Xenograft Model in Nude Mice. Vehicles = 0.5% (w/v) methylcellulose; 0.5% (w/v) methylcellulose, 0.2% Tween 80^{™}. Fitted group tumor volumes after oral administration of Compound 1 dosed PO, QD or Cetuximab dosed IP, BIW alone or in combination for 21 days are depicted. Dose levels are expressed as free-base equivalents.

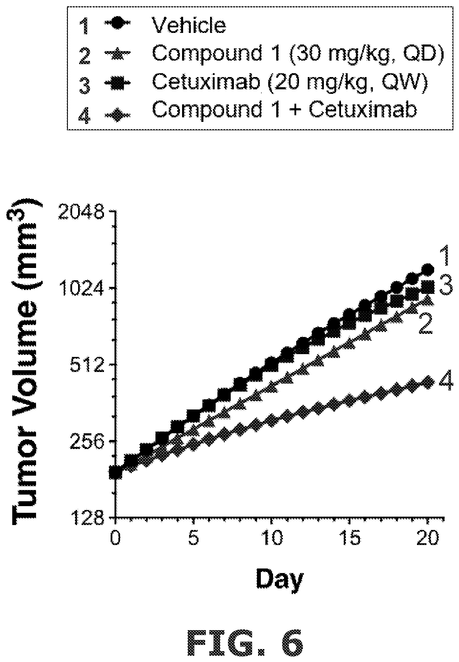

FIG. 6 illustrates the effect of Compound 1 and Cetuximab Dosed Alone or in Combination in CR6927 Colorectal Patient-Derived Xenograft Model in Nude Mice. Vehicles = 0.5% (w/v) methylcellulose; 0.5% (w/v) methylcellulose, 0.2% Tween 80^{™}. Fitted group tumor volumes after oral administration of Compound 1 dosed PO, QD or Cetuximab dosed IP, BIW alone or in combination for 21 days are depicted. Dose levels are expressed as free-base equivalents.

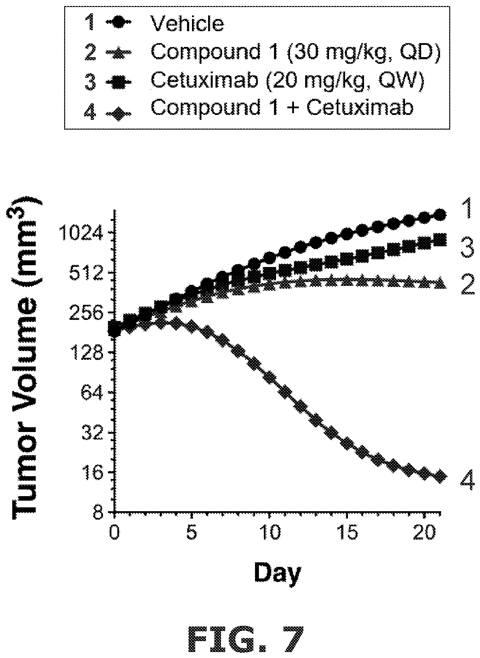

FIG. 7 illustrates the effect of Compound 1 and Cetuximab Dosed Alone or in Combination in CR2528 Colorectal Patient-Derived Xenograft Model in Nude Mice. Vehicles = 0.5% (w/v) methylcellulose; 0.5% (w/v) methylcellulose, 0.2% Tween 80^{™}. Fitted group tumor volumes after oral administration of Compound 1 dosed PO, QD or Cetuximab dosed IP, BIW alone or in combination for 21 days are depicted. Dose levels are expressed as free-base equivalents.

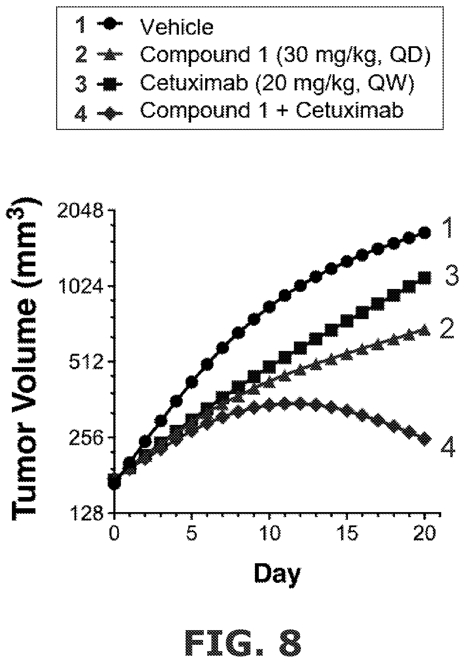

FIG. 8 shows the Effect of Compound 1 and Cetuximab Dosed Alone or in Combination in CR1451 Colorectal Patient-Derived Xenograft Model in Nude Mice. Vehicles = 0.5% (w/v) methylcellulose; 0.5% (w/v) methylcellulose, 0.2% Tween 80^{™}. Fitted group tumor volumes after oral administration of Compound 1 dosed PO, QD or Cetuximab dosed IP, BIW alone or in combination for 21 days are depicted. Dose levels are expressed as free-base equivalents.

### DETAILED DESCRIPTION

### DEFINITIONS

Unless defined otherwise, all technical and scientific terms used herein have the same meaning as commonly understood by those of ordinary skill in the art to which the invention belongs. See, e.g., Singleton et al., DICTIONARY OF MICROBIOLOGY AND MOLECULAR BIOLOGY 2nd ed., J. Wiley & Sons (New York, NY 1994); Sambrook et al., MOLECULAR CLONING, A LABORATORY MANUAL, Cold Springs Harbor Press (Cold Springs Harbor, NY 1989). Any methods, devices and materials similar or equivalent to those described herein can be used in the practice of this invention.

The following definitions are provided to facilitate understanding of certain terms used frequently herein and are not meant to limit the scope of the present disclosure.

As used herein, and unless otherwise specified, the terms "about" and "approximately," when referring to doses, amounts, or weight percents of ingredients of a composition or a dosage form, mean a dose, amount, or weight percent that is recognized by one of ordinary skill in the art to provide a pharmacological effect equivalent to that obtained from the specified dose, amount, or weight percent. The equivalent dose, amount, or weight percent can be within 30%, 20%, 15%, 10%, 5%, 1%, or less of the specified dose, amount, or weight percent.

A "KRas^{G12C} inhibitor" as used herein refers to a covalent inhibitor that specifically binds to a mutant KRas protein comprising a Gly to Cys mutation at a position corresponding to residue 12.

"Compound 1" refers to a compound having structure: 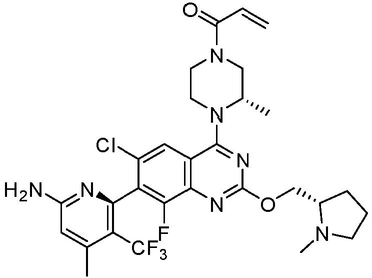 having the chemical name 1-((*S*)-4-((*R*)-7-(6-amino-4-methyl-3-(trifluoromethyl)pyridin-2-yl)-6-chloro-8-fluoro-2-(((*S*)-1-methylpyrrolidin-2-yl)methoxy)quinazolin-4-yl)-3-methylpiperazin-1-yl)prop-2-en-1-one. In one embodiment, Compound 1 is an adipate salt.

"Erlotinib" refers to a compound having structure: 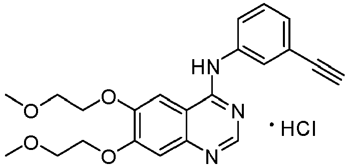 and having the chemical name: *N*-(3-ethynylphenyl)-6,7-bis(2-methoxyethoxy)-4-quinazolinamine. In one embodiment, erlotinib is marketed under the tradename TARCEVA^{®}.

"Gefitinib" refers to a compound having structure: 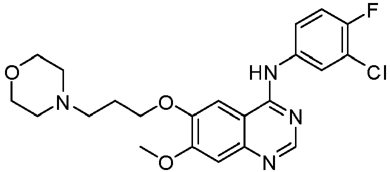 and having the chemical name: 4-Quinazolinamine *N*-(3-chloro-4-fluorophenyl)-7-methoxy-6-[3-(4-morpholinyl) propoxy]. In one embodiment, gefitinib is marketed under the tradename IRESSA^{®}.

"Osimertinib" refers to a compound having structure: 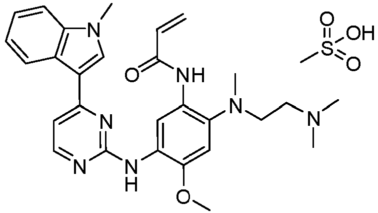 and having the chemical name: *N*-(2-{2-dimethylaminoethyl-methylamino}-4-methoxy-5-{[4-(1-methylindol-3-yl)pyrimidin-2-yl]amino}phenyl)prop-2-enamide mesylate salt. In one embodiment, osimertinib is marketed under the tradename TAGRISSO^{®}.

"Afatinib" refers to a compound having structure: 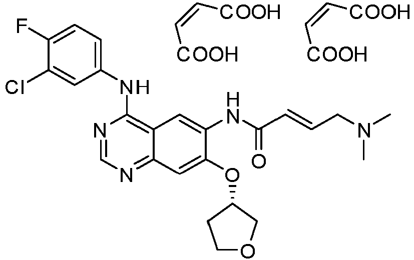 and having the chemical name: 2-butenamide, *N*-[4-[(3-chloro-4-fluorophenyl)amino]-7-[[(3S)-tetrahydro-3-furanyl]oxy]-6-quinazolinyl]-4-(dimethylamino)-,(2*E*)-, (2*Z*)-2-butenedioate (1:2). In one embodiment, afatinib is marketed under the tradename GILOTRIF^{®}.

"Dacomitinib" refers to a compound having structure: 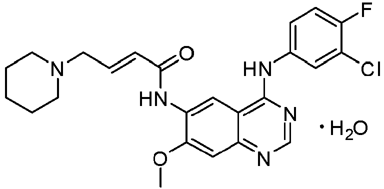 and having the chemical name: (2*E*)-*N*-{4-[(3-Chloro-4-fluorophenyl)amino]-7-methoxyquinazolin-6-yl}-4-(piperidin-1-yl)but-2-enamide monohydrate. In one embodiment, dacomitinib is marketed under the tradename VIZIMPRO^{®}.

The term "pharmaceutically acceptable" refers to molecular entities and compositions that do not produce an adverse, allergic or other untoward reaction when administered to an animal, such as, for example, a human, as appropriate.

Compounds of the invention may be in the form of a salt, such as a pharmaceutically acceptable salt. "Pharmaceutically acceptable salts" include both acid and base addition salts. "Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, carbonic acid, phosphoric acid and the like, and organic acids may be selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, malonic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, benzenesulfonic acid, p-toluenesulfonic acid, salicylic acid and the like. In one embodiment, the salt is formed with adipic acid.

The term "pharmaceutically acceptable base addition salts" include those derived from inorganic bases such as sodium, potassium, lithium, ammonium, calcium, magnesium, iron, zinc, copper, manganese, aluminum salts and the like. Particular base addition salts are the ammonium, potassium, sodium, calcium and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. Particular organic non-toxic bases include isopropylamine, diethylamine, ethanolamine, tromethamine, dicyclohexylamine, choline, and caffeine.

In some embodiments, a salt is selected from a hydrochloride, hydrobromide, trifluoroacetate, sulfate, phosphate, acetate, fumarate, maleate, tartrate, lactate, citrate, pyruvate, succinate, oxalate, methanesulfonate, p-toluenesulfonate, bisulfate, benzenesulfonate, ethanesulfonate, malonate, xinafoate, ascorbate, oleate, nicotinate, saccharinate, adipate, formate, glycolate, palmitate, L-lactate, D-lactate, aspartate, malate, L-tartrate, D-tartrate, stearate, furoate (e.g., 2-furoate or 3-furoate), napadisylate (naphthalene-1,5-disulfonate or naphthalene-1-(sulfonic acid)-5-sulfonate), edisylate (ethane-1,2-disulfonate or ethane-1-(sulfonic acid)-2-sulfonate), isothionate (2-hydroxyethylsulfonate), 2-mesitylenesulfonate, 2-naphthalenesulfonate, 2,5-dichlorobenzenesulfonate, D-mandelate, L-mandelate, cinnamate, benzoate, adipate, esylate, malonate, mesitylate (2-mesitylenesulfonate), napsylate (2-naphthalenesulfonate), camsylate (camphor-10-sulfonate, for example (1S)-(+)-10-camphorsulfonic acid salt), glutamate, glutarate, hippurate (2-(benzoylamino)acetate), orotate, xylate (p-xylene-2-sulfonate), and pamoic (2,2'-dihydroxy-1,1'-dinaphthylmethane-3,3'-dicarboxylate).

The terms "inhibiting" and "reducing," or any variation of these terms, includes any measurable decrease or complete inhibition to achieve a desired result. For example, there may be a decrease of about, at most about, or at least about 5%, 10%, 15%, 20%, 25%, 30%, 35%, 40%, 45%, 50%, 55%, 60%, 65%, 70%, 75%, 80%, 85%, 90%, 95%, 99%, or more, or any range derivable therein, reduction of activity compared to normal.

The terms "EGFR antagonist" , "EGFR-inhibitor" , or "EGFR-specific antagonist" are used interchangeably herein and refer to a molecule capable of binding to EGFR, reducing EGFR expression levels, or neutralizing, blocking, inhibiting, abrogating, reducing, or interfering with EGFR biological activities. Included as EGFR-specific antagonists useful in the methods of the invention are compounds provided herein as well as polypeptides that specifically bind to EGFR, anti-EGFR antibodies and antigen-binding fragments thereof, and molecules and derivatives which bind specifically to EGFR thereby sequestering its binding to one or more receptors or ligands. EGFR-specific antagonists also include antagonist variants of EGFR polypeptides, antisense nucleobase oligomers complementary to at least a fragment of a nucleic acid molecule encoding a EGFR polypeptide; small RNAs complementary to at least a fragment of a nucleic acid molecule encoding a EGFR polypeptide; ribozymes that target EGFR; peptibodies to EGFR; and EGFR aptamers. Thus, the term "EGFR activities" specifically includes EGFR mediated biological activities of EGFR. In certain embodiments, the EGFR antagonist reduces or inhibits, by at least 10%, 20%, 30%, 40%, 50%, 60%, 70%, 80%, 90% or more, the expression level or biological activity of EGFR.

An "anti-EGFR antibody" is an EGFR-inhibitor as defined herein and is an antibody that binds to EGFR with sufficient affinity and specificity. In certain embodiments, the antibody will have a sufficiently high binding affinity for EGFR, for example, the antibody may bind hEGFR with a K_{d} value of between 100 nM-1 pM. Antibody affinities may be determined, e.g., by a surface plasmon resonance-based assay (such as the BIAcore^{®} assay as described in PCT Application Publication No. WO2005/012359); enzyme-linked immunoabsorbent assay (ELISA); and competition assays (e.g. radioimmunoassays (RIAs)).

In certain embodiments, the EGFR-inhibitor (e.g. a compound described herein or anti-EGFR antibody described herein) can be used as a therapeutic agent in targeting and interfering with diseases or conditions wherein the EGFR activity is involved. Also, EGFR-inhibitor may be subjected to other biological activity assays, e.g., in order to evaluate its effectiveness as a therapeutic. Such assays are known in the art and, in instances of an anti-EGFR antibody, depend on the target antigen and intended use for the antibody. In one embodiment, an anti-EGFR antibody is a monoclonal antibody. In another embodiment, an anti-EGFR antibody is a recombinant humanized anti-EGFR monoclonal antibody.

"Cetuximab" as used herein refers to a recombinant, human/mouse chimeric monoclonal antibody that binds specifically to the extracellular domain of the human epidermal growth factor receptor (EGFR). Cetuximab is composed of the Fv regions of a murine anti-EGFR antibody with human IgG1 heavy and kappa light chain constant regions and has an approximate molecular weight of 152 kDa. Cetuximab is produced in mammalian (murine myeloma) cell culture. In one embodiment, cetuximab is marketed under the tradename ERBITUX^{®}.

"Panitumumab" as used herein refers to a human IgG2 kappa monoclonal antibody with an approximate molecular weight of 147 kDa that is produced in genetically engineered mammalian (Chinese hamster ovary) cells. Panitumumab binds specifically to EGFR on both normal and tumor cells, and competitively inhibits the binding of ligands for EGFR. In one embodiment, panitumumab is marketed under the tradename VECTIBIX^{®}.

The term "cancer" refers to a disease caused by an uncontrolled division of abnormal cells in a part of the body. In one embodiment, the cancer is lung cancer. In another embodiment, the cancer is NSCLC. In another embodiment, the cancer is colorectal cancer (e.g. metastatic CRC). In another embodiment, the cancer is pancreatic cancer. "Cancer" as used herein, refers to cancer characterized as having a KRas^{G12C} mutation.

As used herein, "treating" comprises treatment with an effective amount of a therapeutic agent (e.g., EGFR-inhibitor or Compound 1) or combination of therapeutic agents (e.g., EGFR-inhibitor and Compound 1). In one embodiment, treating refers to treatment with an effective amount of Compound 1 or a pharmaceutically acceptable salt thereof and erlotinib. In one embodiment, treating refers to treatment with an effective amount of Compound 1 or a pharmaceutically acceptable salt thereof and cetuximab. The treatment may be first-line treatment (e.g., the patient may be previously untreated or not have received prior systemic therapy), or second line or later treatment. For example, a patient is successfully "treated" if one or more symptoms associated with a cancer described herein are mitigated or eliminated, including, but are not limited to, reducing the proliferation of (or destroying) cancerous cells, decreasing symptoms resulting from the disease, increasing the quality of life of those suffering from the disease, decreasing the dose of other medications required to treat the disease, and/or prolonging survival of patients.

The term "delaying progression" of a disease refers to deferring, hindering, slowing, retarding, stabilizing, and/or postponing development of a cancer described herein. This delay can be of varying lengths of time, depending on the history of the cancer described herein and/or patient being treated. As is evident to one skilled in the art, a sufficient or significant delay can, in effect, encompass prevention, in that the patient does not develop the cancer.

Herein, an "effective amount" refers to the amount of a therapeutic agent described herein (e.g., EGFR-inhibitor and/or Compound 1) that achieves a therapeutic result. In some examples, the effective amount of a therapeutic agent or a combination of therapeutic agents is the amount of the agent or of the combination of agents that achieves a clinical endpoint as provided herein. In one embodiment, an effective amount refers to the amount of Compound 1 or a pharmaceutically acceptable salt thereof and the amount of erlotinib. In one embodiment, an effective amount refers to the amount of Compound 1 or a pharmaceutically acceptable salt thereof and the amount of cetuximab. An effective amount herein may vary according to factors such as the disease state, age, sex, and weight of the patient, and the ability of the agent to elicit a desired response in the patient. An effective amount is also one in which any toxic or detrimental effects of the treatment are outweighed by the therapeutically beneficial effects. In some embodiments, an effective amount of the drug may have the effect in reducing the number of cancer cells; reducing the tumor size; inhibiting (i.e., slow or stop) cancer cell infiltration into peripheral organs; inhibit (i.e., slow or stop) tumor metastasis; inhibiting (i.e., slow or stop) tumor growth; and/or relieving one or more of the symptoms associated with the disease. An effective amount can be administered in one or more administrations. An effective amount of drug, compound, pharmaceutical composition, or combination therapy described herein can be an amount sufficient to accomplish therapeutic treatment either directly or indirectly.

"Objective response rate" or "ORR" refers the percentage of patients with a confirmed complete response or partial response on two consecutive occasions ≥ 4 weeks apart, as determined by the investigator according to RECIST v1.1.

"Duration of response" or "DOR" refers to the time from the first occurrence of a documented objective response to disease progression, as determined by the investigator according to RECIST v1.1, or death from any cause, whichever occurs first.

"Progression free survival" or "PFS" refers to the time from enrollment to the date of the first recorded occurrence of disease progression, as determined by the investigator using RECIST v1.1 or death from any cause, whichever occurs first.

As used herein, "complete response" and "CR" refers to disappearance of all target lesions and (if applicable) normalization of tumor marker level.

As used herein, "partial response" and "PR" refers to persistence of one or more non-target lesions and/or (if applicable) maintenance of tumor marker level above the normal limits. A PR can also refer to ≥ 30% decrease in sum of diameters of target lesions, in the absence of CR, new lesions, and unequivocal progression in non-target lesions.

An "administration period" or "cycle" refers to a period of time comprising administration of one or more agents described herein ( Compound 1 and EGFR-inhibitor) and an optional period of time comprising no administration of one or more of the agents described herein. For example, a cycle can be 21 days in total and include administration of one or more agents described herein ( Compound 1 and EGFR-inhibitor) each day of the cycle. In another example, a cycle can be 28 days in total length and include administration of one or more agents described herein ( Compound 1 and EGFR-inhibitor) for 21 days and a rest period of 7 days. A "rest period" refers to a period of time where at least one of the agents described herein ( Compound 1 and EGFR-inhibitor) are not administered. In one embodiment, a rest period refers to a period of time where none of the agents described herein ( Compound 1 and EGFR-inhibitor) are administered. A rest period as provided herein can in some instances include administration of another agent that is not Compound 1 or EGFR-inhibitor. In such instances, administration of another agent during a rest period should not interfere or detriment administration of an agent described herein. In one instance, cycle as used herein refers to 21 day cycles without a rest period.

A "dosing regimen" refers to an administration period of the agents described herein comprising one or more cycles, where each cycle can include administration of the agents described herein at different times or in different amounts.

"QD" refers to administration of an agent described herein once daily.

"BID" refers to administration of an agent described herein twice daily.

"Q1W" refers to administration of an agent described herein once every week.

"PO" refers to oral administration of an agent described herein.

"IV" refers to intravenous administration of any agent described herein.

A graded adverse event refers to the severity grading scale as established for by NCI CTCAE. In one embodiment, the adverse event is graded in accordance with the table below.

| Grade | Severity |
|---|---|
| 1 | Mild; asymptomatic or mild symptoms; clinical or diagnostic observations only; or intervention not indicated |
| 2 | Moderate; minimal, local, or non-invasive intervention indicated; or limiting age-appropriate instrumental activities of daily living ^{a} |
| 3 | Severe or medically significant, but not immediately life-threatening; hospitalization or prolongation of hospitalization indicated; disabling; or limiting self-care activities of daily living ^{b, c} |
| 4 | Life-threatening consequences or urgent intervention indicated ^{d} |
| 5 | Death related to adverse event ^{d} |

The term "patient" refers to a human patient. A patient may be an adult.

The term "antibody" herein specifically covers monoclonal antibodies (including full-length monoclonal antibodies), polyclonal antibodies, multispecific antibodies (e.g., bispecific antibodies), and antibody fragments so long as they exhibit the desired biological activity. In one instance, the antibody is a full-length monoclonal antibody.

The term IgG "isotype" or "subclass" as used herein is meant any of the subclasses of immunoglobulins defined by the chemical and antigenic characteristics of their constant regions.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, γ, ε, γ, and µ, respectively. The subunit structures and three-dimensional configurations of different classes of immunoglobulins are well known and described generally in, for example, Abbas et al. Cellular and Mol. Immunology, 4th ed. (W.B. Saunders, Co., 2000). An antibody may be part of a larger fusion molecule, formed by covalent or non-covalent association of the antibody with one or more other proteins or peptides.

The terms "full-length antibody," "intact antibody," and "whole antibody" are used herein interchangeably to refer to an antibody in its substantially intact form, not antibody fragments as defined below. The terms refer to an antibody comprising an Fc region.

The term "Fc region" herein is used to define a C-terminal region of an immunoglobulin heavy chain that contains at least a portion of the constant region. The term includes native sequence Fc regions and variant Fc regions. In one aspect, a human IgG heavy chain Fc region extends from Cys226, or from Pro230, to the carboxyl-terminus of the heavy chain. However, antibodies produced by host cells may undergo post-translational cleavage of one or more, particularly one or two, amino acids from the C-terminus of the heavy chain. Therefore, an antibody produced by a host cell by expression of a specific nucleic acid molecule encoding a full-length heavy chain may include the full-length heavy chain, or it may include a cleaved variant of the full-length heavy chain. This may be the case where the final two C-terminal amino acids of the heavy chain are glycine (G446) and lysine (K447). Therefore, the C-terminal lysine (Lys447), or the C-terminal glycine (Gly446) and lysine (Lys447), of the Fc region may or may not be present. Amino acid sequences of heavy chains including an Fc region are denoted herein without the C-terminal lysine (Lys447) if not indicated otherwise. In one aspect, a heavy chain including an Fc region as specified herein, comprised in an antibody disclosed herein, comprises an additional C-terminal glycinelysine dipeptide (G446 and K447). In one aspect, a heavy chain including an Fc region as specified herein, comprised in an antibody disclosed herein, comprises an additional C-terminal glycine residue (G446). In one aspect, a heavy chain including an Fc region as specified herein, comprised in an antibody disclosed herein, comprises an additional C-terminal lysine residue (K447). In one embodiment, the Fc region contains a single amino acid substitution N297A of the heavy chain. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

A "naked antibody" refers to an antibody that is not conjugated to a heterologous moiety (e.g., a cytotoxic moiety) or radiolabel. The naked antibody may be present in a pharmaceutical composition.

"Antibody fragments" comprise a portion of an intact antibody, preferably comprising the antigen-binding region thereof. In some instances, the antibody fragment described herein is an antigen-binding fragment. Examples of antibody fragments include Fab, Fab', F(ab')₂, and Fv fragments; diabodies; linear antibodies; single-chain antibody molecules (e.g., scFvs); and multispecific antibodies formed from antibody fragments.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies in accordance with the present invention may be made by a variety of techniques, including but not limited to the hybridoma method, recombinant DNA methods, phage-display methods, and methods utilizing transgenic animals containing all or part of the human immunoglobulin loci.

The term "hypervariable region" or "HVR" as used herein refers to each of the regions of an antibody variable domain which are hypervariable in sequence and which determine antigen binding specificity, for example "complementarity determining regions" ("CDRs").

Generally, antibodies comprise six CDRs: three in the VH (CDR-H1, CDR-H2, CDR-H3), and three in the VL (CDR-L1, CDR-L2, CDR-L3). Exemplary CDRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991)); and
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)).

Unless otherwise indicated, the CDRs are determined according to Kabat et al., supra. One of skill in the art will understand that the CDR designations can also be determined according to Chothia, supra, McCallum, supra, or any other scientifically accepted nomenclature system.

"Framework" or "FR" refers to variable domain residues other than complementary determining regions (CDRs). The FR of a variable domain generally consists of four FR domains: FR1, FR2, FR3, and FR4. Accordingly, the CDR and FR sequences generally appear in the following sequence in VH (or VL): FR1-CDR-H1(CDR-L1)-FR2- CDR-H2(CDR-L2)-FR3- CDR-H3(CDR-L3)-FR4.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., supra. Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g., residues 82a, 82b, and 82c, etc., according to Kabat) after heavy chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1-107 of the light chain and residues 1-113 of the heavy chain) (e.g., Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., supra). The "EU index as in Kabat" refers to the residue numbering of the human IgG1 EU antibody.

The term "package insert" is used to refer to instructions customarily included in commercial packages of therapeutic products, that contain information about the indications, usage, dosage, administration, combination therapy, contraindications and/or warnings concerning the use of such therapeutic products.

As used herein, "in combination with" refers to administration of one treatment modality in addition to another treatment modality, for example, a treatment regimen that includes administration of an EGFR-inhibitor described herein (e.g., erlotinib or cetuximab) and Compound 1 or a pharmaceutically acceptable salt thereof. As such, "in combination with" refers to administration of one treatment modality before, during, or after administration of the other treatment modality to the patient.

A drug that is administered "concurrently" with one or more other drugs is administered during the same treatment cycle, on the same day of treatment, as the one or more other drugs, and, optionally, at the same time as the one or more other drugs. For instance, for cancer therapies given every 3 weeks, the concurrently administered drugs are each administered on day 1 of a 3 week cycle.

### COMBINATION THERAPIES

Provided herein are combination therapies (compositions) comprising Compound 1 or a pharmaceutically acceptable salt thereof (e.g. Compound 1 adipate) and an EGFR-inhibitor described herein. In one embodiment, is a combination therapy comprising Compound 1 or a pharmaceutically acceptable salt thereof (e.g. Compound 1 adipate) and gefitinib. In another embodiment, is a combination therapy comprising Compound 1 or a pharmaceutically acceptable salt thereof (e.g. Compound 1 adipate) and osimertinib. In another embodiment, is a combination therapy comprising Compound 1 or a pharmaceutically acceptable salt thereof (e.g. Compound 1 adipate) and dacomitinib. In still another embodiment, is a combination therapy comprising Compound 1 or a pharmaceutically acceptable salt thereof (e.g. Compound 1 adipate) and afatinib. In still another embodiment, is a combination therapy comprising Compound 1 or a pharmaceutically acceptable salt thereof (e.g. Compound 1 adipate) and panitumumab. In one preferred embodiment, is a combination therapy comprising Compound 1 or a pharmaceutically acceptable salt thereof (e.g. Compound 1 adipate) and erlotinib or cetuximab. In another preferred embodiment, the combination therapy comprises erlotinib. In another such embodiment, the combination therapy comprises cetuximab.

Further provided herein are combination therapies (compositions) comprising Compound 1 or a pharmaceutically acceptable salt thereof (e.g. Compound 1 adipate) and an EGFR-inhibitor compound ( gefitinib, erlotinib, osimertinib, dacomitinib, or afatinib). In one such embodiment, the EGFR-inhibitor is erlotinib.

Further provided herein are combination therapies (compositions) comprising Compound 1 or a pharmaceutically acceptable salt thereof (e.g. Compound 1 adipate) and an anti-EGFR antibody ( panitumumab or cetuximab). In one such embodiment, the anti-EGFR antibody is cetuximab.

In one aspect provided herein is a combination therapy comprising Compound 1 or a pharmaceutically acceptable salt thereof (e.g. Compound 1 adipate) and an EGFR-inhibitor (e.g. erlotinib or cetuximab). In one embodiment, the combination therapies described herein are useful in the treatment of certain solid tumors comprising KRas^{G12C} mutations. In one such embodiment, the combination therapies are useful in the treatment of certain solid tumors comprising KRas^{G12C} mutations where the EGFR-inhibitor is not approved for administration in such tumors.

In one embodiment, the combination therapies described herein are useful in the treatment of certain types of lung cancer as described herein comprising KRas^{G12C} mutations. In one such embodiment, the lung cancer is non-small cell lung cancer (NSCLC) comprising a KRas^{G12C} mutation.

In another embodiment, the combination therapies described herein are useful in the treatment of colorectal cancer comprising a KRas^{G12C} mutation. In one such embodiment, the combination therapies described herein useful in the treatment of colorectal cancer comprising a KRas^{G12C} mutation are administered in combination with one or more additional agents. In another such embodiment, the additional agent is irinotecan. In another such embodiment, the additional agent comprises FOLFIRI (i.e. administration of leucovorin, fluorouracil, and irinotecan). In another such embodiment, the addition agent comprises FOLFOX (i.e. administration of leucovorin, fluorouracil, and oxaliplatin).

In **a** non-claimed aspect, the combination therapies described herein are useful in the treatment of pancreatic cancer comprising a KRas^{G12C} mutation. In one such embodiment, the combination therapies described herein useful in the treatment of pancreatic cancer comprising a KRas^{G12C} mutation are administered in combination with one or more additional agents. In one such embodiment, the additional agent comprises gemcitabine.

In one aspect provided herein is a combination therapy comprising Compound 1 or a pharmaceutically acceptable salt thereof administered QD on days 1-21 of a first 21-day cycle and an EGFR-inhibitor (e.g. erlotinib or cetuximab). In such embodiments, the combination therapies are useful in the treatment of a solid tumor comprising KRas^{G12C} mutations as described herein ( lung cancer, colorectal cancer).

In one aspect provided herein is a combination therapy comprising Compound 1 or a pharmaceutically acceptable salt thereof administered QD on days 1-21 of a first 21-day cycle and erlotinib administered QD on days 1-21 of the first cycle.

In another aspect provided herein is a combination therapy comprising Compound 1 or a pharmaceutically acceptable salt thereof administered QD on days 1-21 of a first 21-day cycle and cetuximab administered Q1W starting on day 1 of the first 21-day cycle.

In one embodiment of the combination therapies described herein, Compound 1 or a pharmaceutically acceptable salt thereof is administered as a fixed dose QD administration. In one embodiment, the administration is oral (PO), where Compound 1 or a pharmaceutically acceptable salt thereof is formulated as a tablet or capsule. In one such embodiment, Compound 1 or a pharmaceutically acceptable salt thereof is formulated (and administered) as a film coated tablet.

In one embodiment of the combination therapies described herein, Compound 1 or a pharmaceutically acceptable salt thereof is administered at an amount of about 5mg-600mg, 5mg-500mg, 5mg-400mg, 5mg-300mg, 5mg-250mg, 5mg-200mg, 5mg-150mg, 5mg-100mg, 5mg-50mg, 5mg-25mg, 25mg-600mg, 25mg-500mg, 25mg-400mg, 25mg-300mg, 25mg-250mg, 25mg-200mg, 25mg-150mg, 25mg-100mg, 25mg-50mg, 50mg-800mg, 50mg-700mg, 50mg-600mg, 50mg-500mg, 50mg-400mg, 50mg-300mg, 50mg-250mg, 50mg-200mg, 50mg-150mg, or 50mg-100mg QD. In another embodiment, Compound 1 or a pharmaceutically acceptable salt thereof is administered at an amount of about 5mg, 25mg, 50mg, 100mg, 150mg, 200mg, 250mg, 300mg, 400mg or 500mg. In another embodiment, Compound 1 or a pharmaceutically acceptable salt thereof is administered at an amount of about 100mg, 200mg, 300mg, 400mg, 500mg, 600mg, 700mg, or 800mg. In one such embodiment, Compound 1 or a pharmaceutically acceptable salt thereof is administered at an amount of about 300-600mg. In another such embodiment, Compound 1 or a pharmaceutically acceptable salt thereof is administered at an amount of about 400mg. In one preferred embodiment, Compound 1 of the combination therapies described herein is administered as an adipate salt. In such embodiments, the amount of Compound 1 or a pharmaceutically acceptable salt thereof is administered as an amount relative to the free-base form.

In one embodiment of the combination therapy described herein, the EGFR-inhibitor is administered in accordance with a package insert.

In one embodiment, the combination therapy described herein comprises erlotinib, where erlotinib is administered at an amount of about 25mg-200mg, 25mg-150mg, 25mg-100mg, or 25mg-50mg. In one embodiment, erlotinib is administered at an amount of about 100mg. In another embodiment, erlotinib is administered at an amount of about 150mg.

In one embodiment, erlotinib is administered as a component of a combination therapy described herein at an amount of 150 mg QD. In another embodiment, erlotinib is administered as a component of a combination therapy described herein at an amount of 100 mg QD. In such embodiments, erlotinib can be administered in combination with Compound 1 or a pharmaceutically acceptable salt thereof in a dosing regimen comprising administration of each agent QD in a 21-day cycle. In one such embodiment, erlotinib is administered at the same time as Compound 1 or a pharmaceutically acceptable salt thereof with water in between doses. In one embodiment, the amount of erlotinib administered in a combination therapy described herein can be reduced. In one embodiment, the amount of erlotinib is reduced in 25 or 50 mg increments.

In another embodiment, the combination therapy described herein comprises cetuximab, where cetuximab is administered at an amount of about 200-400 mg/m². In one embodiment, cetuximab is administered at an amount of about 400 mg/m² as a first/initial dose. In another embodiment, cetuximab is administered at an amount of about 250 mg/m². In one such embodiment, cetuximab is administered at an amount of about 400 mg/m² on Day 1 of the first 21-day cycle and at 250 mg/m² Q1W of the first 21-day cycle.

Also provided herein are combination therapies comprising Compound 1 or a pharmaceutically acceptable salt thereof and gefitinib, where gefitinib is administered at an amount of 250 mg QD for each 21-day cycle.

Further provided herein are combination therapies comprising Compound 1 or a pharmaceutically acceptable salt thereof and osimertinib, where osimertinib is administered at an amount of 80 mg QD for each 21-day cycle.

Further provided herein are combination therapies comprising Compound 1 or a pharmaceutically acceptable salt thereof and dacomitinib, where dacomitinib is administered at an amount of 45 mg QD for each 21-day cycle.

Still further provided herein are combination therapies comprising Compound 1 or a pharmaceutically acceptable salt thereof and afatinib, where afatinib is administered at an amount of 40 mg QD for each 21-day cycle.

Still further provided herein are combination therapies comprising Compound 1 or a pharmaceutically acceptable salt thereof and panitumumab, where panitumumab is administered at an amount of 6 mg/kg Q2W of each 21-day cycle.

In one preferred embodiment, the combination therapies described herein comprise Compound 1 or a pharmaceutically acceptable salt thereof as described herein administered QD and erlotinib, where erlotinib is administered to the patient at a dose of about 150 mg QD. In another preferred embodiment, the combination therapies described herein comprise Compound 1 or a pharmaceutically acceptable salt thereof as described herein administered QD and cetuximab, where cetuximab is administered in an amount of about 400 mg/m² on Day 1 of a first 21-day cycle and at 250 mg/m² Q1W of the first 21-day cycle.

In one embodiment, the combination therapies described herein are used for treating lung cancer comprising a KRas^{G12C} mutation. In one such embodiment, the combination therapy comprises Compound 1 or a pharmaceutically acceptable salt thereof (e.g. Compound 1 adipate) and an EGRF inhibitor compound selected from the group consisting of erlotinib, gefitinib, osimertinib, dacomitinib, or afatinib. In another such embodiment, the combination therapy comprises Compound 1 or a pharmaceutically acceptable salt thereof (e.g. Compound 1 adipate) and erlotinib, where the combination therapy is for treating lung cancer comprising a KRas^{G12C} mutation as described herein. In such embodiments, the lung cancer is non-small cell lung carcinoma (NSCLC). In one such embodiment, the lung cancer is adenocarcinoma, squamous-cell lung carcinoma or large-cell lung carcinoma. The lung cancer can be stage I or II lung cancer. In one embodiment, the lung cancer is stage III or IV lung cancer.

In another embodiment are combination therapies useful in the treatment of lung cancer comprising a KRas^{G12C} mutation where the combination therapy comprises Compound 1 or a pharmaceutically acceptable salt thereof (e.g. Compound 1 adipate) where Compound 1 is administered QD on days 1-21 of a first 21-day cycle and erlotinib, where erlotinib is administered QD on days 1-21 of the first 21-day cycle. In one preferred embodiment, the lung cancer is NSCLC (e.g. metastatic NSCLC).

In still another embodiment is a combination therapy useful in the treatment of lung cancer comprising a KRas^{G12C} mutation where the combination therapy comprises Compound 1 or a pharmaceutically acceptable salt thereof (e.g. Compound 1 adipate) where Compound 1 is administered QD at an amount of about 50mg-500mg on days 1-21 of a first 21-day cycle and erlotinib, where erlotinib is administered QD at an amount of about 150 mg on days 1-21 of the first 21-day cycle. In one preferred embodiment, the lung cancer is NSCLC. In one embodiment, erlotinib is administered according to a package insert.

In still another embodiment are combination therapies described herein that are useful in the treatment of CRC comprising a KRas^{G12C} mutation. In one particular embodiment, the combination therapy comprises Compound 1 or a pharmaceutically acceptable salt thereof (e.g. Compound 1 adipate) and an anti-EGFR antibody selected from cetuximab or panitumumab, where the combination therapy is for treating CRC comprising a KRas^{G12C} mutation as described herein. In one preferred embodiment, is a combination therapy comprising Compound 1 or a pharmaceutically acceptable salt thereof (e.g. Compound 1 adipate) and cetuximab, where the combination therapy is for treating CRC comprising a KRas^{G12C} mutation as described herein. In one such embodiment, the CRC is metastatic CRC (mCRC). In one embodiment, the combination therapy is for first-line use treatment of CRC comprising a KRas^{G12C} mutation. In another embodiment, the combination therapy is for secondline treatment of CRC comprising a KRas^{G12C} mutation. In one such embodiment, the patient has previously progressed disease having had been previously treated with a KRas^{G12C} inhibitor.

In such embodiments where the combination therapy comprises Compound 1 or a pharmaceutically acceptable salt thereof (e.g. Compound 1 adipate) and cetuximab, and is useful for treating CRC comprising a KRas^{G12C} mutation, a patient described herein may also be administered a FOLFIRI regimen or irinotecan.

In such embodiments where the combination therapy comprises Compound 1 or a pharmaceutically acceptable salt thereof (e.g. Compound 1 adipate) and an anti-EGFR antibody (e.g. panitumumab), and is useful for treating CRC comprising a KRas^{G12C} mutation, a patient described herein may also be administered a FOLFOX regimen.

In another embodiment are combination therapies useful in the treatment of CRC comprising a KRas^{G12C} mutation where the combination therapy comprises Compound 1 or a pharmaceutically acceptable salt thereof (e.g. Compound 1 adipate) where Compound 1 is administered QD on days 1-21 of a first 21-day cycle and cetuximab, where cetuximab is administered in an amount of about 400 mg/m² on Day 1 of a first 21-day cycle and at 250 mg/m² Q1W of the first 21-day cycle. In one preferred embodiment, the CRC is metastatic CRC (mCRC).

In another embodiment are combination therapies useful in the treatment of CRC comprising a KRas^{G12C} mutation where the combination therapy comprises Compound 1 or a pharmaceutically acceptable salt thereof (e.g. Compound 1 adipate) where Compound 1 is administered QD at an amount of about 50mg-500mg on days 1-21 of a first 21-day cycle and cetuximab, where cetuximab is administered in an amount of about 400 mg/m² on Day 1 of a first 21-day cycle and at 250 mg/m² Q1W of the first 21-day cycle. In one preferred embodiment, the CRC is metastatic CRC (mCRC). In one embodiment, cetuximab is administered according to a package insert.

In a non-claimed aspect, the combination therapies described herein are used for treating pancreatic cancer comprising a KRas^{G12C} mutation. In one particular aspect, the combination therapy comprises Compound 1 or a pharmaceutically acceptable salt thereof (e.g. Compound 1 adipate) and erlotinib, where the combination therapy is for treating pancreatic cancer comprising a KRas^{G12C} mutation as described herein.

In one such aspect, the combination therapy comprises Compound 1 or a pharmaceutically acceptable salt thereof (e.g. Compound 1 adipate) where Compound 1 is administered QD on days 1-21 of a first 21-day cycle and erlotinib is administered QD on days 1-21 of the first 21-day cycle.

In another such embodiment, the combination therapy comprises Compound 1 or a pharmaceutically acceptable salt thereof (e.g. Compound 1 adipate) where Compound 1 is administered QD at an amount of about 50mg-500mg on days 1-21 of a first 21-day cycle and erlotinib is administered QD at an amount of 100mg or 150mg on days 1-21 of the first 21-day cycle. In one such embodiment, erlotinib is administered at an amount of about 150 mg QD as described herein. In another such embodiment, erlotinib is administered at an amount of about 100 mg QD as described herein. In one embodiment, erlotinib is administered according to a package insert.

### METHODS OF TREATMENT

Also provided herein are methods of treating a solid tumor comprising a KRas^{G12C} mutation in a patient having such a solid tumor described herein (lung cancer or CRC). In one embodiment, is a method of treating lung cancer, or CRC comprising a KRas^{G12C} mutation in a patient having such a solid tumor, the method comprising administering to the patient an effective amount of a combination therapy comprising Compound 1 or a pharmaceutically acceptable salt thereof (e.g. Compound 1 adipate) and an EGFR-inhibitor described herein (an EGFR-inhibitor compound selected from the group consisting of erlotinib, gefitinib, osimertinib, dacomitinib, or afatinib or an anti-EGFR antibody comprising panitumumab or cetuximab). In one embodiment, is a method of treating lung cancer, or CRC comprising a KRas^{G12C} mutation in a patient having such a solid tumor, the method comprising administering to the patient an effective amount of a combination therapy comprising Compound 1 or a pharmaceutically acceptable salt thereof (e.g. Compound 1 adipate) and erlotinib or cetuximab.

In one aspect provided herein is a method of treating lung cancer comprising a KRas^{G12C} mutation in a patient having such a lung cancer, the method comprising administering to the patient an effective amount of a combination therapy comprising Compound 1 or a pharmaceutically acceptable salt thereof (e.g. Compound 1 adipate) and an EGFR-inhibitor compound selected from the group consisting of erlotinib, gefitinib, osimertinib, dacomitinib, or afatinib. In one aspect provided herein is a method of treating lung cancer mediated by a KRas^{G12C} mutation in a patient having such a lung cancer, the method comprising administering to the patient an effective amount of a combination therapy comprising Compound 1 or a pharmaceutically acceptable salt thereof (e.g. Compound 1 adipate) and erlotinib.

In one embodiment of the methods provided herein, the lung cancer is non-small cell lung carcinoma (NSCLC). In another embodiment of the methods provided herein, the lung cancer is adenocarcinoma, squamous-cell lung carcinoma or large-cell lung carcinoma. In one such embodiment, the cancer is lung adenocarcinoma. In another such embodiment, the lung cancer is a small cell lung carcinoma. In another embodiment, the lung cancer is small cell lung carcinoma. In still another embodiment, the lung cancer is glandular tumors, carcinoid tumors or undifferentiated carcinomas. The lung cancer can be stage I or II lung cancer. In one embodiment, the lung cancer is stage III or IV lung cancer.

Also provided herein is a method of treating NSCLC comprising a KRas^{G12C} mutation in a patient having such a cancer, where the method comprises administering to the patient an effective amount of a combination therapy as described herein comprising a dosing regimen comprising: (i) administering an effective amount of Compound 1 or a pharmaceutically acceptable salt thereof QD on days 1-21 of a first 21-day cycle; and (ii) administering an effective amount of erlotinib QD on days 1-21 of the first 21-day cycle. In one embodiment of the method provided herein, the method is for treating adenocarcinoma. In one embodiment of the method provided herein, the method comprises 2 or more cycles. In one such embodiment, the method is for treating first-line NSCLC.

Also provided herein is a method of treating NSCLC comprising a KRas^{G12C} mutation in a patient having such a cancer, where the method comprises administering to the patient an effective amount of a combination therapy as described herein comprising a dosing regimen comprising: (i) administering 50mg-500 mg of Compound 1 or a pharmaceutically acceptable salt thereof QD on days 1-21 of a first 21-day cycle; and (ii) administering about 150 mg erlotinib QD on days 1-21 of the first 21-day cycle.

In another aspect provided herein is a method treating CRC comprising a KRas^{G12C} mutation in a patient having CRC, the method comprising administering to the patient an effective amount of a combination therapy comprising Compound 1 or a pharmaceutically acceptable salt thereof (e.g. Compound 1 adipate) and an anti-EGFR antibody described herein (panitumumab or cetuximab). In another embodiment of the methods provided herein is a method treating CRC comprising a KRas^{G12C} mutation in a patient having CRC, the method comprising administering to the patient an effective amount of a combination therapy comprising Compound 1 or a pharmaceutically acceptable salt thereof (e.g. Compound 1 adipate) and cetuximab.

Also provided herein is a method of treating CRC comprising a KRas^{G12C} mutation in a patient having such a cancer, where the method comprises administering to the patient an effective amount of a combination therapy as described herein comprising a dosing regimen comprising: (i) administering an effective amount of Compound 1 or a pharmaceutically acceptable salt thereof QD on days 1-21 of a first 21-day cycle; and (ii) administering an effective amount of cetuximab Q1W starting on day 1 of the first 21-day cycle. In one such embodiment, 250 or 400 mg/m² as described herein. In one embodiment of the method provided herein, the method comprises 2 or more cycles.

Also provided herein is a method of treating CRC comprising a KRas^{G12C} mutation in a patient having such a cancer, where the method comprises administering to the patient an effective amount of a combination therapy as described herein comprising a dosing regimen comprising: (i) administering 50mg-500 mg of Compound 1 or a pharmaceutically acceptable salt thereof QD on days 1-21 of a first 21-day cycle; and (ii) administering about 400 mg/m² cetuximab on day 1 of the first 21 day cycle following by administering about 250 mg/m² cetuximab Q1W thereafter.

In one embodiment of such methods for treating CRC comprising a KRas^{G12C} mutation, such methods further comprise administering to the patient an effective amount of FOLFIRI or irinotecan as described herein.

Also provided herein and not claimed is a method of treating pancreatic cancer comprising a KRas^{G12C} mutation in a patient having pancreatic cancer, the method comprising administering to the patient an effective amount of a combination therapy comprising Compound 1 or a pharmaceutically acceptable salt thereof (e.g. Compound 1 adipate) and erlotinib.

In another non-claimed aspect, is a method of treating pancreatic cancer comprising a KRas^{G12C} mutation in a patient having such a cancer, where the method comprises administering to the patient an effective amount of a combination therapy as described herein comprising a dosing regimen comprising: (i) administering an effective amount of Compound 1 or a pharmaceutically acceptable salt thereof QD on days 1-21 of a first 21-day cycle; and (ii) administering an effective amount of erlotinib QD on days 1-21 of the first 21-day cycle. In one such embodiment, erlotinib is administered at an amount of about 100 mg or 150 mg as described herein. In one embodiment, erlotinib is administered at an amount of 100 mg. In another such embodiment, Compound 1 or a pharmaceutically acceptable salt thereof is administered at an amount of about 50mg-500mg as described herein.

In one embodiment of the methods described herein, Compound 1 or a pharmaceutically acceptable salt thereof is administered at an amount of about 5mg-600mg, 5mg-500mg, 5mg-400mg, 5mg-300mg, 5mg-250mg, 5mg-200mg, 5mg-150mg, 5mg-100mg, 5mg-50mg, 5mg-25mg, 25mg-600mg, 25mg-500mg, 25mg-400mg, 25mg-300mg, 25mg-250mg, 25mg-200mg, 25mg-150mg, 25mg-100mg, 25mg-50mg, 50mg-800mg, 50mg-700mg, 50mg-600mg, 50mg-500mg, 50mg-400mg, 50mg-300mg, 50mg-250mg, 50mg-200mg, 50mg-150mg, or 50mg-100mg QD. In another embodiment, Compound 1 or a pharmaceutically acceptable salt thereof is administered at an amount of about 5mg, 25mg, 50mg, 100mg, 150mg, 200mg, 250mg, 300mg, 400mg or 500mg. In another embodiment, Compound 1 or a pharmaceutically acceptable salt thereof is administered at an amount of about 100mg, 200mg, 300mg, 400mg, 500mg, 600mg, 700mg, or 800mg. In one such embodiment, Compound 1 or a pharmaceutically acceptable salt thereof is administered at an amount of about 300-600mg. In another such embodiment, Compound 1 or a pharmaceutically acceptable salt thereof is administered at an amount of about 400mg. In one preferred embodiment, Compound 1 of the combination therapies described herein is administered as an adipate salt. In such embodiments, the amount of Compound 1 or a pharmaceutically acceptable salt thereof is administered as an amount relative to the free-base form.

The methods provided herein can include administration of a combination therapy described herein as part of a dosing regimen. In such one embodiment, the dosing regimen comprises one or more cycles. In another embodiment, the dosing regimen comprises at least 2 cycles. In another embodiment, the dosing regimen comprises 2-3 cycles. In another aspect provided herein is the dosing regimen comprises 2, 3, 4, 5, 6, 8, 10, 12, 16, 18, 20, 24, 30, 36, 42, 48, 54, 60, 66, or 72 cycles. In still another embodiment, dosing regimen comprises about 2-72, 2-66, 2-60, 2-54, 2-48, 2-42, 2-36, 2-30, 2-24, 2-18, 2-12, or 2-6 cycles. In one embodiment, the dosing regimen includes administration of a combination therapy as described herein in any number of cycles until the desired response (e.g. PFS, OS, ORR, and/or DOR) reaches a desired outcome (e.g. increase in PFS, OS, ORR, and/or DOR compared to a control described herein). In another embodiment, the dosing regimen includes administration of a combination therapy as described herein in any number of cycles until toxicity develops or the patient otherwise experiences one or more adverse events (AEs) that prevents further administration. In still another embodiment, the dosing regimen includes administration of a combination therapy as described herein in any number of cycles until disease progression.

In one embodiment of the methods described herein, a patient is administered a total of 1 to 50 doses of an anti-EGFR antibody, e.g., 1 to 50 doses, 1 to 45 doses, 1 to 40 doses, 1 to 35 doses, 1 to 30 doses, 1 to 25 doses, 1 to 20 doses, 1 to 15 doses, 1 to 10 doses, 1 to 5 doses, 2 to 50 doses, 2 to 45 doses, 2 to 40 doses, 2 to 35 doses, 2 to 30 doses, 2 to 25 doses, 2 to 20 doses, 2 to 15 doses, 2 to 10 doses, 2 to 5 doses, 3 to 50 doses, 3 to 45 doses, 3 to 40 doses, 3 to 35 doses, 3 to 30 doses, 3 to 25 doses, 3 to 20 doses, 3 to 15 doses, 3 to 10 doses, 3 to 5 doses, 4 to 50 doses, 4 to 45 doses, 4 to 40 doses, 4 to 35 doses, 4 to 30 doses, 4 to 25 doses, 4 to 20 doses, 4 to 15 doses, 4 to 10 doses, 4 to 5 doses, 5 to 50 doses, 5 to 45 doses, 5 to 40 doses, 5 to 35 doses, 5 to 30 doses, 5 to 25 doses, 5 to 20 doses, 5 to 15 doses, 5 to 10 doses, 1 to 50 doses, 1 to 45 doses, 1 to 40 doses, 1 to 35 doses, 1 to 30 doses, 1 to 25 doses, 1 to 20 doses, 1 to 15 doses, 1 to 10 doses, 1 to 8 doses, 1 to 6 doses, 1 to 5 doses, 10 to 50 doses, 10 to 45 doses, 10 to 40 doses, 10 to 35 doses, 10 to 30 doses, 10 to 25 doses, or 10 to 20 doses. In one such embodiment, a patient is administered a total of 1 to 10 doses of an anti-EGFR antibody (e.g. cetuximab). In another such embodiment, a patient is administered a total of 5, 6, 7, 8, 9, or 10 doses of an anti-EGFR antibody (e.g. cetuximab). In one preferred embodiment, the doses of an anti-EGFR antibody (e.g. cetuximab) are administered intravenously.

In certain embodiments, the therapeutic agents of the combination therapies described herein (e.g. Compound 1 or a pharmaceutically acceptable salt thereof and erlotinib or cetuximab) may be administered in any suitable manner known in the art. For example, the EGFR-inhibitor (e.g. erlotinib or cetuximab) may be administered sequentially (on different days) or concurrently (on the same day or during the same treatment cycle) as Compound 1 or a pharmaceutically acceptable salt thereof. In one embodiment, the EGFR-inhibitor (e.g. erlotinib or cetuximab) is administered after administration of Compound 1 or a pharmaceutically acceptable salt thereof. In some instances, the EGFR-inhibitor (e.g. erlotinib or cetuximab) is administered after and on the same day as administration of Compound 1 or a pharmaceutically acceptable salt thereof. In one embodiment, the EGFR-inhibitor (e.g. erlotinib or cetuximab) may be administered after administration of Compound 1 or a pharmaceutically acceptable salt thereof on the same day. For example, Compound 1 or a pharmaceutically acceptable salt thereof can be administered on Day 1 of each cycle prior to administration of the EGFR-inhibitor (e.g. erlotinib or cetuximab) on Day 1 of each cycle, where Compound 1 or a pharmaceutically acceptable salt thereof is then administered QD for the next 20 days of the 21-day cycle.

In a preferred embodiment, cetuximab is administered intravenously after Compound 1 or a pharmaceutically acceptable salt thereof (e.g. about 120 minutes). If the first infusion is tolerated, the second administration of cetuximab is administered IV over 60 minutes ± 10 min. In some examples, cetuximab is administered as an intravenous push or bolus.

Also provided herein are methods for treating lung cancer comprising a KRas^{G12C} mutation in a patient having such a cancer, where the method comprises administering to the patient a treatment regimen comprising an effective amount of Compound 1 or a pharmaceutically acceptable salt thereof (e.g. adipate salt) and an EGFR-inhibitor compound selected from the group consisting of erlotinib, gefitinib, osimertinib, dacomitinib, or afatinib (e.g. erlotinib). In one embodiment of such methods, Compound 1 is an adipate salt and the EGFR-inhibitor compound is erlotinib. In another embodiment of such methods, Compound 1 or a pharmaceutically acceptable salt thereof is administered QD as described herein and in an amount as described herein (e.g. 50mg-500mg). In another embodiment of such methods, erlotinib is administered QD as described herein and in an amount as described herein (e.g. 150 mg). In such methods, Compound 1 or a pharmaceutically acceptable salt thereof and the EGFR-inhibitor can be administered as described herein. In such methods, the lung cancer can be NSCLC comprising a KRas^{G12C} mutation.

Also provided herein are methods for treating CRC comprising a KRas^{G12C} mutation in a patient having such a cancer, where the method comprises administering to the patient a treatment regimen comprising an effective amount of Compound 1 or a pharmaceutically acceptable salt thereof (e.g. adipate salt) and an anti-EGFR antibody described herein (e.g., cetuximab). In one embodiment of such methods, Compound 1 is an adipate salt and the anti-EGFR antibody described herein is cetuximab. In another embodiment of such methods, Compound 1 or a pharmaceutically acceptable salt thereof is administered QD as described herein and in an amount as described herein (e.g. 50mg-500mg). In another embodiment of such methods, cetuximab is administered at an amount of about 400 mg/m² cetuximab on day 1 of the first 21 day cycle following by administering about 250 mg/m² cetuximab Q1W thereafter. In such methods, Compound 1 or a pharmaceutically acceptable salt thereof and cetuximab can be administered as described herein.

In another embodiment, is a method for treating CRC comprising a KRas^{G12C} mutation in a patient having such a cancer, where the method comprises administering to the patient a treatment regimen comprising: (i) administering about 50mg-500mg of Compound 1 or a pharmaceutically acceptable salt thereof (e.g. adipate salt) QD on days 1-21 during a first 21-day cycle; and (ii) administering about 400 mg/m² cetuximab on day 1 of the first 21-day cycle followed by administering about 250 mg/m² cetuximab Q1W thereafter.

Also provided herein and not claimed are methods for treating pancreatic cancer comprising a KRas^{G12C} mutation in a patient having such a cancer, where the method comprises administering to the patient a treatment regimen comprising an effective amount of Compound 1 or a pharmaceutically acceptable salt thereof (e.g. adipate salt) and an EGFR-inhibitor described herein (e.g., erlotinib). In one embodiment of such methods, Compound 1 is an adipate salt and the EGFR-inhibitor described herein is erlotinib. In another embodiment of such methods, Compound 1 or a pharmaceutically acceptable salt thereof is administered QD as described herein and in an amount as described herein (e.g. 50mg-500mg). In another embodiment of such methods, erlotinib is administered QD as described herein at an amount of about 100 mg or 150 mg. In such methods, Compound 1 or a pharmaceutically acceptable salt thereof and erlotinib can be administered as described herein.

In another non-claimed aspect, is a method of treating pancreatic cancer comprising a KRas^{G12C} mutation in a patient having such a cancer, where the method comprises administering to the patient a treatment regimen comprising (i) administering about 50mg-500mg of Compound 1 or a pharmaceutically acceptable salt thereof (e.g. adipate salt) QD on days 1-21 to the patient during a first 21-day cycle and (ii) administering 100 mg or 150 mg erlotinib QD on days 1-21 to the patient during the first 21-day cycle.

In some instances, the treatment regimen includes administration of one or more additional therapies where the additional therapy is one or more side-effect limiting agents (e.g., agents intended to lessen the occurrence and/or severity of side effects of treatment, such as anti-nausea agents, a corticosteroid (e.g., prednisone or an equivalent, e.g., at a dose of 1-2 mg/kg/day), hormone replacement medicine(s), and the like).

A patient as provided herein, must be evaluated and have a confirmed test result for a KRas^{G12C} mutation as set forth herein. In one embodiment, a patient described herein has a confirmed test result for a KRas^{G12C} mutation for CRC. In one such embodiment, the patient has been previously treated with one or more prior therapies. A patient described herein having diagnosed NSCLC and a confirmed test result for a KRas^{G12C} mutation must not have a known concomitant second oncogenic driver (e.g., for NSCLC: sensitizing EGFR mutations, ALK rearrangement, ROS1 rearrangement, BRAF V600E mutation, NTRK fusions, RET fusions; or for adenocarcinoma of the colon or rectum: BRAF V600E mutation, ERBB2 amplification). In one such embodiment, the patient has been previously treated with one or more prior therapies. In one embodiment, such second oncogenic drivers are determined using NGS (e.g. by the Foundation Medicine, Inc. (FMI) NGS assay).

In one embodiment of the methods provided herein, where a patient described herein is treated with a combination therapy comprising cetuximab, such a patient has experienced disease progression or intolerance to at least one prior chemotherapy regimen (e.g., FOLFOX, FOLFIRI, FOLFOXIRI ± bevacizumab).

In another embodiment of the methods provided herein, where a patient described herein is treated with a combination therapy comprising erlotinib, such a patient has experienced disease progression or intolerance to at least 1 prior systemic therapy (e.g. single-agent or combination therapy with an investigational or approved PD-L1/PD-1 inhibitor).

In one embodiment, a patient described herein has received prior treatment with a KRas^{G12C} specific inhibitor.

In another embodiment, a patient described herein has not received treatment with chemotherapy, immunotherapy, or biologic therapy as anti-cancer therapy within 3 weeks prior to administration of a combination therapy described herein, or endocrine therapy within 2 weeks prior to administration of a combination therapy described herein, except for the following:
(a) hormonal therapy with gonadotropin-releasing hormone (GnRH) agonists or antagonists for endocrine sensitive cancers (e.g., prostate, endometrial, hormone receptor-positive breast cancer);
(b) kinase inhibitors, approved by regulatory authorities, may be used up to 2 weeks prior to administration of a combination therapy described herein, provided any drug-related toxicity has completely resolved; or
(c) treatment with an investigational agent within 3 weeks or five half-lives prior to administration of a combination therapy described herein, whichever is shorter.

In another embodiment, a patient described herein has not received radiation therapy (other than palliative radiation to bony metastases and radiation to CNS metastases as described above) as cancer therapy within 4 weeks prior to initiation of administration of a combination therapy described herein. In still another embodiment, a patient described herein has not received palliative radiation to bony metastases within 2 weeks prior to administration of a combination therapy described herein.

In another embodiment, a patient described herein does not have a history of idiopathic pulmonary fibrosis, organizing pneumonia (e.g., bronchiolitis obliterans), drug-induced pneumonitis, or idiopathic pneumonitis, or evidence of active pneumonitis on screening chest computed tomography (CT) scan.

Further provided herein is the use (UL1) of a combination therapy described herein comprising Compound 1 or a pharmaceutically acceptable salt thereof and an EGFR-inhibitor compound selected from the group consisting of erlotinib, gefitinib, osimertinib, dacomitinib, or afatinib for the treatment of lung cancer as described herein. In one embodiment, is a use (UL2) of a combination therapy described herein comprising Compound 1 or a pharmaceutically acceptable salt thereof and erlotinib for the treatment of lung cancer as described herein. In one such embodiment, the lung cancer is NSCLC.

Further provided herein is the use (UL3) of a combination therapy described herein comprising Compound 1 or a pharmaceutically acceptable salt thereof and erlotinib for the treatment of lung cancer as described herein comprising a dosing regimen comprising: (i) administering Compound 1 or a pharmaceutically acceptable salt thereof QD on days 1-21 of a first 21-day cycle; and (ii) administering erlotinib QD on days 1-21 of the first 21-day cycle. In one such embodiment, Compound 1 or a pharmaceutically acceptable salt thereof is administered at an amount of about 50-500 mg. In another such embodiment, erlotinib is administered at an amount of about 150mg.

Further provided herein is the use (UL4) of a combination therapy described herein comprising Compound 1 or a pharmaceutically acceptable salt thereof and erlotinib for the treatment of lung cancer as described herein comprising a dosing regimen comprising: (i) administering about 50-500 mg Compound 1 or a pharmaceutically acceptable salt thereof QD on days 1-21 of a first 21-day cycle; and (ii) administering about 150 mg erlotinib QD on days 1-21 of the first 21-day cycle. In one such embodiment, the dosing regimen includes 2 or more cycles as described herein.

Further provided herein is the use (UL5) of a combination therapy described herein comprising Compound 1 or a pharmaceutically acceptable salt thereof and an EGFR-inhibitor compound selected from the group consisting of erlotinib, gefitinib, osimertinib, dacomitinib, or afatinib for the manufacture of a medicament for the treatment of lung cancer as described herein. In one such embodiment, the EGFR-inhibitor is erlotinib.

Further provided herein is the use (UL6) of a combination therapy described herein comprising Compound 1 or a pharmaceutically acceptable salt thereof and erlotinib for the manufacture of a medicament for the treatment of lung cancer as described herein comprising a dosing regimen comprising: (i) administering Compound 1 or a pharmaceutically acceptable salt thereof QD on days 1-21 of a first 21-day cycle; and (ii) administering erlotinib QD on days 1-21 of the first 21-day cycle. In one such embodiment, Compound 1 or a pharmaceutically acceptable salt thereof is administered at an amount of about 50-500 mg. In another such embodiment, erlotinib is administered at an amount of about 150 mg.

Further provided herein is the use (UL7) of a combination therapy described herein comprising Compound 1 or a pharmaceutically acceptable salt thereof and erlotinib for the manufacture of a medicament for the treatment of lung cancer as described herein comprising a dosing regimen comprising: (i) administering about 50-500 mg Compound 1 or a pharmaceutically acceptable salt thereof QD on days 1-21 of a first 21-day cycle; and (ii) administering about 150 mg erlotinib QD on days 1-21 of the first 21-day cycle. In one such embodiment, the dosing regimen includes 2 or more cycles as described herein.

In such embodiments of the uses described herein, the lung cancer can be NSCLC. In another such embodiment of the uses described herein, a patient described herein is diagnosed with NSCLC mediated by a KRas^{G12C} mutation.

Further provided herein is the use (UC1) of a combination therapy described herein comprising Compound 1 or a pharmaceutically acceptable salt thereof and an anti-EGFR antibody selected from the group consisting of cetuximab or panitumumab for the treatment of CRC as described herein. In one embodiment, is a use (UC2) of a combination therapy described herein comprising Compound 1 or a pharmaceutically acceptable salt thereof and cetuximab for the treatment of CRC as described herein. In one such embodiment, the CRC is mCRC.

Further provided herein is the use (UC3) of a combination therapy described herein comprising Compound 1 or a pharmaceutically acceptable salt thereof and cetuximab for the treatment of CRC as described herein comprising a dosing regimen comprising: (i) administering Compound 1 or a pharmaceutically acceptable salt thereof QD on days 1-21 of a first 21-day cycle; and (ii) administering about 400 mg/m² cetuximab on day 1 of the 21-day cycle. In one such embodiment, Compound 1 or a pharmaceutically acceptable salt thereof is administered at an amount of about 50-500 mg. In another such embodiment, cetuximab is administered at an amount of about 400 mg/m² on day 1 of the first 21 day cycle following by administering cetuximab at an amount of about 250 mg/m² Q1W thereafter.

Further provided herein is the use (UC4) of a combination therapy described herein comprising Compound 1 or a pharmaceutically acceptable salt thereof and cetuximab for the treatment of lung cancer as described herein comprising a dosing regimen comprising: (i) administering about 50-500 mg Compound 1 or a pharmaceutically acceptable salt thereof QD on days 1-21 of a first 21-day cycle; and (ii) administering about 400 mg/m² cetuximab on day 1 of the first 21 day cycle following by administering about 250 mg/m² cetuximab Q1W thereafter.

Further provided herein is the use (UC5) of a combination therapy described herein comprising Compound 1 or a pharmaceutically acceptable salt thereof and an an anti-EGFR antibody selected from the group consisting of cetuximab or panitumumab for the manufacture of a medicament for the treatment of CRC as described herein. In one such embodiment, the anti-EGFR antibody is cetuximab.

In such embodiments of the uses described herein, a patient described herein is diagnosed with CRC mediated by a KRas^{G12C} mutation.

Further provided herein is the use (UC6) of a combination therapy described herein comprising Compound 1 or a pharmaceutically acceptable salt thereof and cetuximab for the manufacture of a medicament for the treatment of CRC as described herein comprising a dosing regimen comprising: (i) administering Compound 1 or a pharmaceutically acceptable salt thereof QD on days 1-21 of a first 21-day cycle; and (ii) administering cetuximab Q1W starting on day 1 of the first 21-day cycle. In one such embodiment, Compound 1 or a pharmaceutically acceptable salt thereof is administered at an amount of about 50-500 mg. In another such embodiment, cetuximab is administered at an amount of about 400 mg/m² cetuximab on day 1 of the first 21 day cycle following by administering cetuximab at an amount of about 250 mg/m² Q1W thereafter.

Further provided herein is the use (UC6) of a combination therapy described herein comprising Compound 1 or a pharmaceutically acceptable salt thereof and cetuximab for the manufacture of a medicament for the treatment of CRC as described herein comprising a dosing regimen comprising: (i) administering about 50-500 mg Compound 1 or a pharmaceutically acceptable salt thereof QD on days 1-21 of a first 21-day cycle; and (ii) administering about 400 mg/m² cetuximab on day 1 of the first 21 day cycle following by administering about 250 mg/m² cetuximab Q1W thereafter. In one such embodiment, the dosing regimen includes 2 or more cycles as described herein.

Further provided herein and not claimed is the use (UP1) of a combination therapy described herein comprising Compound 1 or a pharmaceutically acceptable salt thereof and **erlotinib** for the treatment of pancreatic cancer as described herein.

Further provided herein and not claimed is the use (UP2) of a combination therapy described herein comprising Compound 1 or a pharmaceutically acceptable salt thereof and erlotinib for the treatment of pancreatic cancer as described herein comprising a dosing regimen comprising: (i) administering Compound 1 or a pharmaceutically acceptable salt thereof QD on days 1-21 of a first 21-day cycle; and (ii) administering erlotinib QD on days 1-21 of the first 21-day cycle. In one such embodiment, Compound 1 or a pharmaceutically acceptable salt thereof is administered at an amount of about 50-500 mg. In another such embodiment, erlotinib is administered at an amount of about 100mg.

Further provided herein and not claimed is the use (UP3) of a combination therapy described herein comprising Compound 1 or a pharmaceutically acceptable salt thereof and erlotinib for the treatment of pancreatic cancer as described herein comprising a dosing regimen comprising: (i) administering about 50-500 mg Compound 1 or a pharmaceutically acceptable salt thereof QD on days 1-21 of a first 21-day cycle; and (ii) administering about 100 mg erlotinib QD on days 1-21 of the first 21-day cycle. In one such embodiment, the dosing regimen includes 2 or more cycles as described herein.

Further provided herein and not claimed is the use (UP4) of a combination therapy described herein comprising Compound 1 or a pharmaceutically acceptable salt thereof and erlotinib for the manufacture of a medicament for the treatment of pancreatic cancer as described herein.

Further provided herein and not claimed is the use (UP5) of a combination therapy described herein comprising Compound 1 or a pharmaceutically acceptable salt thereof and erlotinib for the manufacture of a medicament for the treatment of pancreatic cancer as described herein comprising a dosing regimen comprising: (i) administering Compound 1 or a pharmaceutically acceptable salt thereof QD on days 1-21 of a first 21-day cycle; and (ii) administering erlotinib QD on days 1-21 of the first 21-day cycle. In one such embodiment, Compound 1 or a pharmaceutically acceptable salt thereof is administered at an amount of about 50-500 mg. In another such embodiment, erlotinib is administered at an amount of about 100mg.

Further provided herein and not claimed is the use (UP6) of a combination therapy described herein comprising Compound 1 or a pharmaceutically acceptable salt thereof and erlotinib for the manufacture of a medicament for the treatment of pancreatic cancer as described herein comprising a dosing regimen comprising: (i) administering about 50-500 mg Compound 1 or a pharmaceutically acceptable salt thereof QD on days 1-21 of a first 21-day cycle; and (ii) administering about 100mg erlotinib QD on days 1-21 of the first 21-day cycle. In one such embodiment, the dosing regimen includes 2 or more cycles as described herein.

The development of combination treatments poses challenges including, for example, the selection of agents for combination therapy that may lead to improved efficacy while maintaining acceptable toxicity. One particular challenge is the need to distinguish the incremental toxicity of the combination. In one embodiment of the methods described herein the combination therapy described herein (e.g. Compound 1 or a pharmaceutically acceptable salt thereof and erlotinib or cetuximab) is administered in a dosing regimen comprising a staggered dosing schedule. In one such embodiment, the patient has a reduced number or grade of adverse events (AEs) comparable to a control (e.g. SOC therapy, treatment with one agent described herein (e.g. Compound 1 or erlotinib or cetuximab) alone).

It is generally understood that the when an adverse event occurs, four options exist: (1) continue treatment as-is with optional concomitant therapy; (2) adjust the dose of one or more agents in the dosing regimen; (3) suspend administration of one or more agents in the dosing regimen; or (4) discontinue administration of one or more agents in the dosing regimen. In one embodiment, the amount of Compound 1 is not modified. In another embodiment, the amount of erlotinib administered is not modified. In another embodiment, the amount of cetuximab administered is not modified. In one embodiment, where the administration of erlotinib or cetuximab is interrupted, the next administration of Compound 1 or a pharmaceutically acceptable salt thereof occurs on the same day as administration of erlotinib or cetuximab is resumed. In one embodiment, Compound 1 or a pharmaceutically acceptable salt thereof is administered without food (i.e. a patient should not eat at least 2 hours before and 1 hour after administration). In one such embodiment, administration of cetuximab is at least 20, 30, 45, or 60 minutes after administration of Compound 1 or a pharmaceutically acceptable salt thereof. In another such embodiment, administration of erlotinib is after the administration of Compound 1 or a pharmaceutically acceptable salt thereof.

In one embodiment, a patient described herein experiences gastrointestinal toxicity as an AE at a grade of less than or equal to 2. In one such embodiment, the gastrointestinal toxicity is diarrhea, nausea, or vomitting. In another embodiment, a patient described herein experiences phototoxicity. In such embodiments, the patient should wear sunscreen and protective clothing outdoors.

In one embodiment, a patient described herein administered a combination therapy comprising cetuximab experiences a skin reaction, hypomagnesaemia, or IRRs. In another embodiment, a a patient described herein administered a combination therapy comprising erlotinib experiences cutaneous toxicity, interstitial lung disease (ILD), liver injury, gastrointestinal (GI) fluid loss, GI perforation, or ocular toxicity.

Patients described herein can also be administered concomitant therapies including: (a) anti-seizure medications or warfarin; (b) oral contraceptives or other allowed maintenance therapy; (c) anti-emetics and anti-diarrheal medications provided that such medications should not be administered prophylactically before initial treatment with study drug; (d) pain medications administered per standard clinical practice; (e) bisphosphonate and denosumab therapy for bone metastases or osteopenia/osteoporosis; or (f) multivitamins, calcium, and vitamins C, D, and E supplements.

Patients described herein may not concomitantly take therapies including (1) Strong/moderate CYP3A4 inhibitors (e.g. atazanavir, ritonavir, indinavir, nelfinavir, saquinavir, clarithromycin, telithromycin, erythromycin, troleandomycin, fluconazole, itraconazole, ketoconazole, voriconazole, posaconazole, aprepitant, conivaptan, fluvoxamine, diltiazem, nefazodone, mibefradil, verapamil, and grapefruit juice or grapefruit supplements) or (2) Strong/moderate CYP3A4 inducers (e.g. rifampin, carbamazepine, phenytoin, oxcarbazepine, phenobarbital, efavirenz, nevirapine, etravirine, modafinil, hyperforin (St. John's Wort), and cyproterone).

In another embodiment, a patient described herein is not administered a drug that reduces gastric acid production, such as proton pump inhibitors or H2-receptor antagonists. In another embodiment, patients administered a combination therapy comprising erlotinib should not have chronic use of anti-angiogenic agents and nonsteroidal anti-inflammatory drugs (NSAIDs).

In another embodiment, patients described herein are not administered any of the following therapies:
(a) Any other investigational therapy (excluding Compound 1 or erlotinib or cetuximab) within 3 weeks or five half-lives prior to administration of a combination therapy described herein, whichever is shorter, or during such treatment;
(b) Concomitant therapy intended for the treatment of cancer whether approved by the FDA or experimental, including chemotherapy, radiotherapy, immunotherapy, biologic therapy, herbal therapy, or hormonal therapy except for the following:
   (i) Hormonal therapy with gonadotropin-releasing hormone (GnRH) agonists or antagonists for endocrine sensitive cancers (e.g., prostate, endometrial, hormone receptor-positive breast cancer);
   (ii) Hormone replacement therapy or oral contraception;
(c) Radiotherapy for unequivocal progressive disease with the exception of new brain metastases in the setting of systemic response as follows: patients who have demonstrated control of their systemic disease (defined as having received clinical benefit [i.e., a PR, CR, or SD for ≥3 months]), but who have developed brain metastases that are treatable with radiation, will be allowed to continue to receive therapy with Compound 1 during the study until they either experience systemic progression of their disease and/or further progression in the brain (based on investigator assessments).
(d) Quinidine or other anti-arrhythmic agents; or
(e) Initiation or increased dose of hematopoietic colony-stimulating factors (CSFs; e.g., granulocyte CSF; filgrastim, granulocyte/macrophage CSF; sargramostim, pegfilgrastim, erythropoietin, darbepoetin, and thrombopoietin) from 7 days before Cycle 1, Day 1;

In one embodiment of such methods, the patient is diagnosed with a cancer described herein. In another embodiment of such methods, the sample is a tumor sample taken from the subject. In one such embodiment, the sample is taken before administration of any therapy described herein. In another such embodiment, the sample is taken before administration of at least one agent described herein. In some embodiments, tumor samples can be taken at specified intervals during treatment with a combination therapy described herein to assess treatment.

Determining whether a tumor or cancer comprises a KRas^{G12C} mutation can be undertaken by assessing the nucleotide sequence encoding the K-Ras protein, by assessing the amino acid sequence of the K-Ras protein, or by assessing the characteristics of a putative K-Ras mutant protein. The sequence of wild-type human K-Ras (e.g. Accession No. NP203524) is known in the art. In one such embodiment, a sample from a patient described herein is assessed for a KRas^{G12C} mutation using, for example, immunohistochemistry (IHC) or NGS sequencing.

Further provided herein are methods of treating tumor agnostic cancer comprising a KRas^{G12C} mutation by administering a combination therapy as described herein. In one embodiment of such methods, the method comprises:
(a) determining the absence or presence of a KRas^{G12C} mutation in a sample taken from a patient with a suspected diagnosed cancer; and
(b) administering to the patient a combination therapy as described herein comprising an effective amount of Compound 1 or a pharmaceutically acceptable salt thereof and an EGFR-inhibitor described herein.

In one such embodiment, the EGFR-inhibitor is erlotinib or cetuximab. In one such embodiment, Compound 1 or a pharmaceutically acceptable salt thereof is administered QD at an amount of about 50-500 mg. In another such embodiment, erlotinib is administered QD at an amount of about 100 mg or 150 mg. In still another embodiment, cetuximab is administered at an amount about 400 mg/m² on day 1 of a first 21 day cycle followed by administering cetuximab at an amount of about 250 mg/m² Q1W thereafter.

Further provided herein are methods of treating tumor agnostic cancer comprising a KRas^{G12C} mutation where the method comprises:
(a) determining the absence or presence of a KRas^{G12C} mutation in a sample taken from a patient with a suspected diagnosed cancer; and
(b) administering to the patient a combination therapy as described herein comprising dosing regimen comprising: (i) administering 50mg-500mg Compound 1 or a pharmaceutically acceptable salt thereof QD on days 1-21 of a first 21-day cycle; and (ii) administering 100 or 150 mg erlotinib QD on days 1-21 of the first 21-day cycle.

Further provided herein are methods of treating tumor agnostic cancer comprising a KRas^{G12C} mutation where the method comprises:
(a) determining the absence or presence of a KRas^{G12C} mutation in a sample taken from a patient with a suspected diagnosed cancer; and
(b) administering to the patient a combination therapy as described herein comprising dosing regimen comprising: (i) administering 50mg-500mg Compound 1 or a pharmaceutically acceptable salt thereof QD on days 1-21 of a first 21-day cycle; and (ii) administering about 400 mg/m² cetuximab on day 1 of the first 21 day cycle following by administering about 250 mg/m² cetuximab Q1W thereafter.

In one embodiment of the methods provided herein a patient is diagnosed having a CR following treatment with a combination therapy according to the methods provided herein. In one embodiment of the methods provided herein a patient is diagnosed having a PR following treatment with a combination therapy according to the methods provided herein. In one embodiment of the methods provided herein a patient is diagnosed having SD following treatment with a combination therapy according to the methods provided herein.

Also provided herein are methods of inhibiting tumor growth or producing tumor regression in a patient described herein by administering a combination therapy described herein. In one embodiment provided herein is a method of inhibiting tumor growth in a patient having a cancer described herein by administering a combination therapy comprising Compound 1 or a pharmaceutically acceptable salt thereof and an EGFR-inhibitor described herein (e.g. erlotinib or cetuximab) in one or more 21-day cycles as described herein. In one embodiment provided herein is a method of inhibiting tumor growth in a patient having NSCLC, or CRC as described herein by administering a combination therapy comprising Compound 1 or a pharmaceutically acceptable salt thereof and an EGFR-inhibitor described herein (e.g. erlotinib or cetuximab) in one or more 21-day cycles as described herein.

In one embodiment provided herein is a method of producing or improving tumor regression in a patient having a cancer described herein by administering a combination therapy comprising administering Compound 1 or a pharmaceutically acceptable salt thereof and an EGFR-inhibitor described herein (e.g. erlotinib or cetuximab) in one or more 21-day cycles as described herein. In one embodiment provided herein is a method of producing or improving tumor regression in a patient having NSCLC, or CRC described herein by administering a combination therapy comprising administering Compound 1 or a pharmaceutically acceptable salt thereof and an EGFR-inhibitor described herein (e.g. erlotinib or cetuximab) in one or more 21-day cycles as described herein. KITS

The combination therapies described herein can be provided as a kit comprising one or more of the agents described herein for administration. In one embodiment, the kit includes Compound 1 or a pharmaceutically acceptable salt thereof (e.g. Compound 1 adipate) for administration in combination with an EGFR-inhibitor described herein (e.g. erlotinib or cetuximab) as described herein. In another embodiment, the kit includes Compound 1 or a pharmaceutically acceptable salt thereof (e.g. Compound 1 adipate) packaged together with an EGFR-inhibitor described herein (e.g. erlotinib or cetuximab), where the kit comprises separate formulated dosages of each agent.

Also provided herein is an article of manufacture or a kit comprising Compound 1 or a pharmaceutically acceptable salt thereof (e.g. Compound 1 adipate) and an EGFR-inhibitor described herein (e.g. erlotinib or cetuximab). In some instances, the article of manufacture further comprises package insert comprising instructions for using the EGFR-inhibitor described herein (e.g. erlotinib or cetuximab) to treat or delay progression of a solid tumor (lung cancer, or CRC as described herein). In one such embodiment, the cancer is NSCLC. In one embodiment, the article of manufacture further comprises package insert comprising instructions for using an EGFR-inhibitor described herein (e.g. erlotinib) in combination with Compound 1 or a pharmaceutically acceptable salt thereof (e.g. Compound 1 adipate) to treat or delay progression of NSCLC in a patient. In one embodiment, the article of manufacture further comprises package insert comprising instructions for using an EGFR-inhibitor described herein (e.g. cetuximab) in combination with Compound 1 or a pharmaceutically acceptable salt thereof (e.g. Compound 1 adipate) to treat or delay progression of CRC in a patient.

In some instances, the EGFR-inhibitor described herein (e.g. erlotinib or cetuximab) and Compound 1 or a pharmaceutically acceptable salt thereof (e.g. Compound 1 adipate) are in the same container or separate containers. Suitable containers include, for example, bottles, vials, bags and syringes. The container may be formed from a variety of materials such as glass, plastic (such as polyvinyl chloride or polyolefin), or metal alloy (such as stainless steel or hastelloy). In some instances, the container holds the formulation and the label on, or associated with, the container may indicate directions for use. The article of manufacture or kit may further include other materials desirable from a commercial and user standpoint, including other buffers, diluents, filters, needles, syringes, and package inserts with instructions for use. In some instances, the article of manufacture further includes one or more of another agent (e.g., an additional chemotherapeutic agent or anti-neoplastic agent). Suitable containers for the one or more agents include, for example, bottles, vials, bags and syringes.

Any of the articles of manufacture or kits described herein may include instructions to administer Compound 1 or a pharmaceutically acceptable salt thereof (e.g. Compound 1 adipate) and/or the EGFR-inhibitor described herein (e.g. erlotinib or cetuximab) to a patient in accordance with any of the methods described herein.

### BIOMARKERS

In one embodiment, the alkylation of KRas^{G12C} by Compound 1 or a pharmaceutically acceptable salt thereof is measured in the patient. In one such embodiment, the measurement is performed using a sample and tested for alkylation of KRas^{G12C} as provided herein. In another embodiment, assessment of ctDNA biomarkers (e.g., KRas^{G12C}) from peripheral blood is performed.

In one embodiment, modulation of KRAS/MAPK target genes (e.g., *DUSP6, SPRY4*), pathway components (e.g., pERK, pS6), and related biomarkers (e.g., Ki67) through analysis of paired pre-treatment and on-treatment fresh tumor biopsies is performed.

The following Examples are presented by way of illustration, not limitation.

### EXAMPLES

### Example 1: Combination of Compound 1 and Erlotinib

The Kirsten rat sarcoma viral oncogene homolog (KRAS) gene encodes a GTPase that plays a central role in mediating cell growth and survival signaling. Mutations in KRAS that result in amino acid substitutions at glycine 12 (G12), glycine 13 (G13), and glutamine 61 (Q61) are common in tumors and are associated with tumorigenesis and maintenance of aggressive tumor growth (Der et al. Nature 1983;304(5926):507-13; Parada et al. Nature 1982;297(5866):474-8; Santos et al. Nature 1982;298(5872):343-7; Taparowsky et al. Nature 1982;300(5894):762-5; Capon et al. Nature 1983;304(5926): 507-13). The KRAS^{G12C} mutation is prevalent in non-small cell lung cancer (NSCLC), colorectal cancer, and other tumor types (Prior et al. Cancer Res 2012;72(10):2457-67; Vogelestein et al. Science 2013;339(6127):1546-58).

Compound 1 is an oral anti-cancer therapeutic agent that selectively targets KRAS^{G12C}, resulting in covalent and irreversible inhibition of KRAS^{G12C}. Compound 1 does not target other mutations in KRAS, the wild-type form of KRAS, or other members of the RAS family. Treatment of KRAS^{G12C}-positive cells or tumors with Compound 1 results in decreased KRAS pathway signaling, suppression of cell/tumor cell growth, and induction of apoptosis.

The in vivo anti-tumor efficacy of Compound 1 (50 mg/kg, PO, QD) alone or in combination with erlotinib (50 mg/kg, PO, QD) in the NCI-H2122 (*KRAS^{G12C}*) NSCLC xenograft tumor model was determined. Single agent Compound 1 treatment resulted in tumor stasis (93% tumor growth inhibition (TGI)), whereas single agent treatment with erlotinib resulted in tumor growth inhibition of only 48% TGI. Improved anti-tumor efficacy was observed with combinations of Compound 1 and erlotinib (117% TGI).

Test Material. Compound 1 (free base) was provided as a solution at a concentration of 8.333 mg/mL (expressed as free-base equivalents) in 0.5% *(w*/*v)* methylcellulose. Erlotinib (Tarceva^{™}) was provided as a solution at a concentration of 12.5 mg/mL (expressed as free-base equivalents) in 7.5% Captisol. All concentrations were calculated based on a mean body weight of 25 g for the nude mouse strain used in this study. The vehicle controls were 0.5% *(w*/*v)* methylcellulose and 0.5% *(w*/*v)* methylcellulose/0.2% Tween 80^{™}.Test agents were stored in a refrigerator set to maintain a temperature range of 4°C-7°C. All treatments and vehicle control dosing solutions were prepared once a week for three weeks.

Female nude mice that were 9-10 weeks old were obtained from Charles River Laboratory (Hollister, CA) weighing an average of 24.5 g. The mice were housed in standard rodent micro-isolator cages and were acclimated to study conditions at least 3 days before tumor cell implantation. Only animals that appeared to be healthy and that were free of obvious abnormalities were used for the study.

Human non-small lung carcinoma NCI-H2122 cells were obtained from the American Type Culture Collection (Rockville, MD) and harbor a G12C oncogenic mutation in *K-RAS.* Cells were cultured *in vitro,* harvested in log-phase growth, and resuspended in Hank's Balanced Salt Solution (HBSS) containing Matrigel (BD Biosciences; San Jose, CA) at a 1:1 ratio. The cells were then implanted subcutaneously in the right lateral thorax of 160 nude mice. Each mouse was injected with 10 × 10⁶ cells in a volume of 100 µL. Tumors were monitored until they reached a mean tumor volume of 150-290 mm³. Mice were distributed into ten groups based on tumor volumes with n=10 mice per group. The mean tumor volume across all ten groups was 213 mm³ at the initiation of dosing.

Mice were given vehicles (150 µL 0.5% MC and 100 µL 0.5% MCT), 50 mg/kg Compound 1 (expressed as free-base equivalents), or 50 mg/kg erlotinib. All treatments were administered on a daily basis (QD) orally (PO) by gavage for 21 days. Tumor sizes and mouse body weights were recorded and mice were promptly euthanized when tumor volume exceeded 2000 mm³ or if body weight loss was ≥ 20% of their starting weight.

**Table 1: Study Design**

| No./Sex | Treatment | Dose level (mg/kg)^{a} | No./Sex | Route | Days of Dosing | Dose Conc. (mg/mL)^{a} | Dose Volume (mL/kg) |
|---|---|---|---|---|---|---|---|
| 10/F | Vehicles | 0 (Vehicles) | 10/F | PO | 21 | 0 | 6, 4 |
| 10/F | Compound 1 | 50 | 10/F | PO | 21 | 8.33 | 6 |
| 10/F | Erlotinib | 50 | 10/F | PO | 21 | 12.5 | 4 |
| 10/F | Compound 1 + erlotinib | 50 + 50 | 10/F | PO | 21 | 8.33, 12.5 | 6, 4 |
| Conc. = concentration; PO = orally; QD = once daily. | | | | | | | |
| Note: Vehicle controls were 0.5% (w/v) methylcellulose (100 µ) + 0.5% (w/v) methylcellulose; 0.2% Tween 80^{™} (150 µL). | | | | | | | |
| ^{a}Dose levels and concentrations are expressed as free-base equivalents and were dosed once daily (QD) for 21 days. | | | | | | | |

Tumor volumes were measured in two dimensions (length and width) using Ultra Cal-IV calipers (model 54 - 10 - 111; Fred V. Fowler Co.; Newton, MA) and analyzed using Excel, version 14.2.5 (Microsoft Corporation; Redmond WA). The tumor volume was calculated with the following formula:$Tumor size \left({mm}^{3}\right) = \left(longer measurement \times shorter {measurement}^{2}\right) \times 0.5$

Anti-tumor responses were noted with partial responses (PRs) being defined as a >50% decrease from the initial tumor volume and complete responses (CRs) being defined as a 100% decrease in tumor volume.

Anti-tumor efficacy was assessed in nude mice bearing human NCI-H2122 NSCLC xenografts following treatment with Compound 1 (50 mg/kg, PO, QD) alone compared to single agent erlotinib (50 mg/kg, PO, QD) or when combined. The single agent treatments resulted in tumor growth inhibition (TGI), with Compound 1 resulted in 93% TGI and erlotinib resulted in 48% relative to vehicle controls (see Table 2 and FIG. 1). Improved anti-tumor was observed with combinations of Compound 1 and erlotinib, resulting in 117% TGI and 3/10 partial responses (PRs) (Figure 2).

**Table 2: Anti-Tumor Activity of Compound 1 and Erlotinib Dosed Alone or in Combination in Nude Mice with Human NCI-H2122 NSCLC Xenograft Tumors**

| Group (n = 10) | Treatment | Dose Levels (mg/kg) | TI | PR | CR | % TGI (estimated) | % TGI (lower CI) | % TGI (upper CI) |
|---|---|---|---|---|---|---|---|---|
| 1 | Vehicles | 0 (Vehicles) | 10/10 | 0 | 0 | 0 | 0 | 0 |
| 2 | Compound 1 | 50 | 10/10 | 0 | 0 | 93 | 80 | 100 |
| 3 | Erlotinib | 50 | 10/10 | 0 | 0 | 48 | 24 | 66 |
| 7 | Compound 1 + erlotinib | 50 + 50 | 10/10 | 0 | 0 | 117 | 111 | 125 |

| | | | | | | | | |
|---|---|---|---|---|---|---|---|---|
| CI = confidence interval; CR = complete response; PR = partial response; QD = once daily; TI = tumor incidence. Vehicle = 0.5% (w/v) methylcellulose; 0.5% (w/v) methylcellulose/0.2% Tween 80^{™}. | | | | | | | | |

Combination anti-tumor efficacy studies were performed in the NCI-H2122 human NSCLC xenograft tumor model demonstrating that the KRAS^{G12C} inhibitor, Compound 1, suppresses tumor growth (93% TGI, no PRs) as a single agent. Single agent activity with the EGFR-inhibitor, erlotinib, also resulted in tumor growth inhibition (48% TGI, no PRs). Combination of Compound 1 and erlotinib resulted in improved anti-tumor efficacy (117% TGI, 3/10 PRs). These data demonstrate that combination of the KRAS^{G12C} inhibitor, Compound 1, with erlotinib resulted in improved anti-tumor activity leading to partial tumor regressions in the NCI-H2122 human NSCLC human xenograft tumor model.

### Example 2: Combination of Compound 1 and cetuximab in PDX CR6256 Colon Cancer Xenograft Model in female BALB/c nude Mice.

The *in vivo* therapeutic efficacy combination of Compound 1 and cetuximab was evaluated preclinically in the treatment of subcutaneous PDX CR6256 Colon Cancer Xenograft Model in female BALB/c nude Mice.

Female BALB/c nude mice were housed in standard polysulfone IVC cages. Mice were 5-9 weeks of age at initial inoculation. Compound 1 was administered PO at 30 mg/kg QD for 21 days. Cetuximab was administered intraperitoneal (IP) at 20 mg/kg BIW for 3 weeks.

Tumor fragments from stock mice were harvested and used for inoculation into mice. Each mouse was inoculated subcutaneously in the right rear flank with primary human tumor xenograft model CR6256 tumor fragment (2-3 mm in diameter) for tumor development. After tumor cells inoculation, the animals were checked daily for morbidity and mortality. During routine monitoring, the animals were checked for any effects of tumor growth and treatments on behavior such as mobility, food and water consumption, body weight gain/loss (Body weights would be measured twice per week after randomization), eye/hair matting and any other abnormalities. Mortality and observed clinical signs were recorded for individual animals in detail.

Tumor volumes were measured twice per week after randomization in two dimensions using a caliper, and the volume was expressed in mm³ using the formula: V = (L x W x W)/2, where V is tumor volume, L is tumor length (the longest tumor dimension) and W is tumor width (the longest tumor dimension perpendicular to L). Dosing as well as tumor and body weight measurements were conducted in a Laminar Flow Cabinet. The body weights and tumor volumes were measured by using Study Director^{™} software (version 3.1.399.19).

Tumor growth inhibition (TGI): TGI% is an indication of antitumor activity, and expressed as: TGI (%) =100 x (1-T/C). T and C are the mean tumor volume (or weight) of the treated and control groups, respectively, on a given day.

The *in vivo* anti-tumor efficacy of Compound 1 (30 mg/kg, orally once daily) alone or in combination with cetuximab, was evaluated in the CR6256 (*KRas^{G12C}*) colorectal patient-derived tumor model. Single agent Compound 1 treatment resulted in tumor stasis to regression (108% tumor growth inhibition [TGI]), whereas single agent cetuximab, showed moderate to minor tumor growth inhibition (74%). Combination of Compound 1 with cetuximab, showed improved combination efficacy (133%) relative to the single agents.

**Table 3: Anti-Tumor Activity of Compound 1 and Cetuximab Dosed Alone or in Combination in CR6256 Colorectal Patient-Derived Xenograft Model in Nude Mice**

| Treatment | Dose Levels (mg/kg), Schedule | TI | PR | CR | % TGI (estimated) | % TGI (lower CI) | % TGI (upper CI) |
|---|---|---|---|---|---|---|---|
| Vehicles | 0 (Vehicles) | 10/10 | 0 | 0 | 0 | 0 | 0 |
| Compound 1 | 30, PO, QD | 10/10 | 6 | 1 | 108 | 97 | 121 |
| Cetuximab | 20, IP, BIW | 10/10 | 0 | 0 | 74 | 51 | 89 |
| Compound 1 + Cetuximab | 30 + 20 | 10/10 | 1 | 9 | 133 | 121 | 155 |
| CI = confidence interval; CR = complete response; PR = partial response; | | | | | | | |
| QD = once daily; TI = tumor incidence. | | | | | | | |
| Vehicle = 0.5% (w/v) methylcellulose. | | | | | | | |

### Example 3: Combination of Compound 1 and cetuximab in PDX Cancer Model CR5048 in female NOD-SCID mice.

The in vivo therapeutic efficacy combination of Compound 1 and cetuximab was evaluated preclinically in the treatment of the PDX Cancer Model CR5048 in female NOD-SCID mice. Animals were randomized on Day 0 when average tumor volume reached 185.67 mm³ and dosing was initiated on Day 1. Animals were dosed daily (QD) for 21 days with Compound 1 and BIWx3.5 weeks (7 total doses) with cetuximab, alone and in combination. All animals were terminated 8 hours post last dose (Study Day 21). Animals were measured twice weekly for the duration of the study. At the end of the study, tumors and blood were collected from all animals on study. Tumors were split into half and both pieces were snap frozen in liquid nitrogen in separate tubes. Blood was collected by cardiac puncture and processed to plasma.

The in vivo anti-tumor efficacy of Compound 1 (30 mg/kg, orally, once daily) alone or in combination with cetuximab in the CR5048 (*KRas^{G12C}*) colorectal patient-derived tumor model was measured. Single agent Compound 1 treatment resulted in tumor growth inhibition (90% tumor growth inhibition [TGI]), whereas single agent cetuximab treatment showed moderate to minor tumor growth inhibition (59% TGI). Combination of Compound 1 with cetuximab showed improved combination efficacy (110%) relative to the single agents.

**Table 4: Anti-Tumor Activity of Compound 1 and Cetuximab Dosed Alone or in Combination in CR5048 Colorectal Patient-Derived Xenograft Model in Nude Mice**

| Treatment | Dose Levels (mg/kg), Schedule | TI | PR | CR | % TGI (estimated) | % TGI (lower CI) | % TGI (upper CI) |
|---|---|---|---|---|---|---|---|
| Vehicles | 0 (Vehicles) | 10/10 | 0 | 0 | 0 | 0 | 0 |
| Compound 1 | 30, PO, QD | 10/10 | 0 | 0 | 90 | 78 | 100 |
| Compound 1 + Cetuximab | 30 + 20 | 10/10 | 10 | 0 | 110 | 104 | 118 |
| Cetuximab | 20, IP, BIW | 10/10 | 0 | 0 | 59 | 29 | 77 |
| CI = confidence interval; CR = complete response; PR = partial response; | | | | | | | |
| QD = once daily; TI = tumor incidence. | | | | | | | |
| Vehicle = 0.5% (w/v) methylcellulose. | | | | | | | |

### Example 4: Combination of Compound 1 and cetuximab PDX CR6243 Colon Cancer Xenograft Model in Female BALB/c Nude Mice

The *in vivo* therapeutic efficacy combination of Compound 1 and cetuximab was evaluated preclinically in the treatment of subcutaneous PDX CR6243 Colon Cancer Xenograft Model in female BALB/c nude Mice.

Female BALB/c nude mice were housed in standard polysulfone IVC cages. Mice were 5-9 weeks at initial inoculation. Compound 1 was administered PO at 30 mg/kg QD for 21 days. Cetuximab was administered intraperitoneal (IP) at 20 mg/kg BIW for 3 weeks.

Tumor fragments from stock mice were harvested and used for inoculation into mice. Each mouse was inoculated subcutaneously in the right rear flank with primary human tumor xenograft model CR6243 tumor fragment (2-3 mm in diameter) for tumor development

The randomization started when the mean tumor size reaches approximately 192 mm³. After tumor cells inoculation, the animals were checked daily for morbidity and mortality. During routine monitoring, the animals were checked for any effects of tumor growth and treatments on behavior such as mobility, food and water consumption, body weight gain/loss (Body weights would be measured twice per week after randomization), eye/hair matting and any other abnormalities. Mortality and observed clinical signs were recorded for individual animals in detail.

Tumor volumes were measured twice per week after randomization in two dimensions using a caliper, and the volume was expressed in mm³ using the formula: V = (L x W x W)/2, where V is tumor volume, L is tumor length (the longest tumor dimension) and W is tumor width (the longest tumor dimension perpendicular to L). Dosing as well as tumor and body weight measurements were conducted in a Laminar Flow Cabinet. The body weights and tumor volumes were measured by using Study Director^{™} software (version 3.1.399.19).

Tumor growth inhibition (TGI): TGI% is an indication of antitumor activity, and expressed as: TGI (%) =100 x (1-T/C). T and C are the mean tumor volume (or weight) of the treated and control groups, respectively, on a given day.

The *in vivo* anti-tumor efficacy of Compound 1 (30 mg/kg, orally, once daily) alone or in combination with cetuximab in the CR6243 (KRAS^{G12C}) colorectal patient-derived tumor model. Single agent Compound 1 treatment resulted in tumor stasis to regression (89% tumor growth inhibition [TGI]), whereas single agent cetuximab showed moderate to minor tumor growth inhibition (47% TGI). Combination of Compound 1 with cetuximab showed improved combination efficacy (104%) relative to the single agents.

**Table 5: Anti-Tumor Activity of Compound 1 and Cetuximab Dosed Alone or in Combination in CR6243 Colorectal Patient-Derived Xenograft Model in Nude Mice**

| Treatment | Dose Levels (mg/kg), Schedule | TI | PR | CR | % TGI (estimated) | % TGI (lower CI) | % TGI (upper CI) |
|---|---|---|---|---|---|---|---|
| Vehicles | 0 (Vehicles) | 10/10 | 0 | 0 | 0 | 0 | 0 |
| Compound 1 | 30, PO, QD | 10/10 | 0 | 0 | 89 | 82 | 95 |
| Cetuximab | 20, IP, BIW | 10/10 | 0 | 0 | 47 | 27 | 62 |
| Compound 1 + Cetuximab | 30 + 20 | 10/10 | 5 | 0 | 104 | 100 | 108 |
| CI = confidence interval; CR = complete response; PR = partial response; | | | | | | | |
| QD = once daily; TI = tumor incidence. | | | | | | | |
| Vehicle = 0.5% (w/v) methylcellulose. | | | | | | | |

### Example 5: Combination of Compound 1 and cetuximab in PDX CR6927 Colorectal Cancer Xenograft Model in Female BALB/c Nude Mice

The *in vivo* therapeutic efficacy combination of Compound 1 and cetuximab was evaluated preclinically in the treatment of subcutaneous PDX CR6927 Colon Cancer Xenograft Model in female BALB/c nude Mice.

Female BALB/c nude mice were housed in standard polysulfone IVC cages. Mice were 5-9 weeks at initial inoculation. Compound 1 was administered PO at 30 mg/kg QD for 21 days. Cetuximab was administered intraperitoneal (IP) at 20 mg/kg BIW for 3 weeks.

Tumor fragments from stock mice were harvested and used for inoculation into mice. Each mouse was inoculated subcutaneously in the right rear flank with primary human tumor xenograft model CR6927 tumor fragment (2-3 mm in diameter) for tumor development

The randomization started when the mean tumor size reaches approximately 194 mm³. After tumor cells inoculation, the animals were checked daily for morbidity and mortality. During routine monitoring, the animals were checked for any effects of tumor growth and treatments on behavior such as mobility, food and water consumption, body weight gain/loss (Body weights would be measured twice per week after randomization), eye/hair matting and any other abnormalities. Mortality and observed clinical signs were recorded for individual animals in detail.

Tumor volumes were measured twice per week after randomization in two dimensions using a caliper, and the volume was expressed in mm³ using the formula: V = (L x W x W)/2, where V is tumor volume, L is tumor length (the longest tumor dimension) and W is tumor width (the longest tumor dimension perpendicular to L). Dosing as well as tumor and body weight measurements were conducted in a Laminar Flow Cabinet. The body weights and tumor volumes were measured by using Study Director^{™} software (version 3.1.399.19).

Tumor growth inhibition (TGI): TGI% is an indication of antitumor activity, and expressed as: TGI (%) =100 x (1-T/C). T and C are the mean tumor volume (or weight) of the treated and control groups, respectively, on a given day.

The *in vivo* anti-tumor efficacy of Compound 1 (30 mg/kg, orally, once daily) alone or in combination with cetuximab in the CR6927 (KRAS^{G12C}) colorectal patient-derived tumor model. Single agent Compound 1 and cetuximab anti-tumor (29% and 10% tumor growth inhibition [TGI], respectively). Combination of Compound 1 with cetuximab resulted in improved combination efficacy (70%) relative to the single agents. All doses and combinations tested were tolerated based on minimal changes in body weight and overall animal condition.

**Table 6: Anti-Tumor Activity of Compound 1 and Cetuximab Dosed Alone or in Combination in CR6927 Colorectal Patient-Derived Xenograft Model in Nude Mice**

| Treatment | Dose Levels (mg/kg), Schedule | TI | PR | CR | % TGI (estimated) | % TGI (lower CI) | % TGI (upper CI) |
|---|---|---|---|---|---|---|---|
| Vehicles | 0 (Vehicles) | 10/10 | 0 | 0 | 0 | 0 | 0 |
| Compound 1 | 30, PO, QD | 10/10 | 0 | 0 | 29 | -14 | 56 |
| Cetuximab | 20, IP, BIW | 10/10 | 0 | 0 | 10 | -40 | 44 |
| Compound 1 + Cetuximab | 30 + 20 | 10/10 | 1 | 0 | 70 | 49 | 84 |
| CI = confidence interval; CR = complete response; PR = partial response; | | | | | | | |
| QD = once daily; TI = tumor incidence. | | | | | | | |
| Vehicle = 0.5% (w/v) methylcellulose. | | | | | | | |

### Example 6: Combination of Compound 1 and cetuximab in PDX CR2528 Colorectal Cancer Xenograft Model in Female BALB/c Nude Mice

The *in vivo* therapeutic efficacy combination of Compound 1 and cetuximab was evaluated preclinically in the treatment of subcutaneous PDX CR2528 Colon Cancer Xenograft Model in female BALB/c nude Mice.

Female BALB/c nude mice were housed in standard polysulfone IVC cages. Mice were 8-10 weeks at initial inoculation. Compound 1 was administered PO at 30 mg/kg QD for 21 days. Cetuximab was administered intraperitoneal (IP) at 20 mg/kg BIW for 3 weeks.

Tumor fragments from stock mice were harvested and used for inoculation into mice. Each mouse was inoculated subcutaneously in the right rear flank with primary human tumor xenograft model CR2528 tumor fragment (2-3 mm in diameter) for tumor development

The randomization started when the mean tumor size reaches approximately 202 mm³. After tumor cells inoculation, the animals were checked daily for morbidity and mortality. During routine monitoring, the animals were checked for any effects of tumor growth and treatments on behavior such as mobility, food and water consumption, body weight gain/loss (Body weights would be measured twice per week after randomization), eye/hair matting and any other abnormalities. Mortality and observed clinical signs were recorded for individual animals in detail.

Tumor volumes were measured twice per week after randomization in two dimensions using a caliper, and the volume was expressed in mm³ using the formula: V = (L x W x W)/2, where V is tumor volume, L is tumor length (the longest tumor dimension) and W is tumor width (the longest tumor dimension perpendicular to L). Dosing as well as tumor and body weight measurements were conducted in a Laminar Flow Cabinet. The body weights and tumor volumes were measured by using Study Director^{™} software (version 3.1.399.19).

Tumor growth inhibition (TGI): TGI% is an indication of antitumor activity, and expressed as: TGI (%) =100 x (1-T/C). T and C are the mean tumor volume (or weight) of the treated and control groups, respectively, on a given day.

The *in vivo* anti-tumor efficacy of Compound 1 (30 mg/kg, orally, once daily) alone or in combination with cetuximab in the CR2528 (KRAS^{G12C}) colorectal patient-derived tumor model. Single agent Compound 1 treatment resulted in tumor stasis (65% tumor growth inhibition [TGI]), whereas single agent cetuximab showed modest tumor growth inhibition (40% TGI). Combination of Compound 1 with cetuximab resulted in improved combination efficacy (117% TGI) relative to the single agents. All doses and combinations tested were tolerated based on minimal changes in body weight and overall animal condition.

**Table 7: Anti-Tumor Activity of Compound 1 and Cetuximab Dosed Alone or in Combination in CR2528 Colorectal Patient-Derived Xenograft Model in Nude Mice**

| Treatment | Dose Levels (mg/kg), Schedule | TI | PR | CR | % TGI (estimated) | % TGI (lower CI) | % TGI (upper CI) |
|---|---|---|---|---|---|---|---|
| Vehicles | 0 (Vehicles) | 10/10 | 0 | 0 | 0 | 0 | 0 |
| Compound 1 | 30, PO, QD | 10/10 | 0 | 0 | 65 | 20 | 85 |
| Cetuximab | 20, IP, BIW | 10/10 | 0 | 0 | 40 | -34 | 76 |
| Compound 1 + Cetuximab | 30 + 20 | 10/10 | 2 | 7 | 117 | 108 | 132 |
| CI = confidence interval; CR = complete response; PR = partial response; QD = once daily; TI = tumor incidence. | | | | | | | |
| Vehicle = 0.5% (w/v) methylcellulose. | | | | | | | |

### Example 7: Combination of Compound 1 and cetuximab in PDX CR1451 Colorectal Cancer Xenograft Model in Female BALB/c Nude Mice

The in vivo therapeutic efficacy combination of Compound 1 and cetuximab was evaluated preclinically in the treatment of subcutaneous PDX CR1451 Colon Cancer Xenograft Model in female BALB/c nude Mice.

Female BALB/c nude mice were housed in standard polysulfone IVC cages. Mice were 5-9 weeks at initial inoculation. Compound 1 was administered PO at 30 mg/kg QD for 21 days. Cetuximab was administered intraperitoneal (IP) at 20 mg/kg BIW for 3 weeks.

Tumor fragments from stock mice were harvested and used for inoculation into mice. Each mouse was inoculated subcutaneously in the right rear flank with primary human tumor xenograft model CR1451 tumor fragment (2-3 mm in diameter) for tumor development.

The randomization started when the mean tumor size reaches approximately 182 mm³. After tumor cells inoculation, the animals were checked daily for morbidity and mortality. During routine monitoring, the animals were checked for any effects of tumor growth and treatments on behavior such as mobility, food and water consumption, body weight gain/loss (Body weights would be measured twice per week after randomization), eye/hair matting and any other abnormalities. Mortality and observed clinical signs were recorded for individual animals in detail.

Tumor volumes were measured twice per week after randomization in two dimensions using a caliper, and the volume was expressed in mm³ using the formula: V = (L x W x W)/2, where V is tumor volume, L is tumor length (the longest tumor dimension) and W is tumor width (the longest tumor dimension perpendicular to L). Dosing as well as tumor and body weight measurements were conducted in a Laminar Flow Cabinet. The body weights and tumor volumes were measured by using Study Director^{™} software (version 3.1.399.19).

Tumor growth inhibition (TGI): TGI% is an indication of antitumor activity, and expressed as: TGI (%) =100 x (1-T/C). T and C are the mean tumor volume (or weight) of the treated and control groups, respectively, on a given day.

The *in vivo* anti-tumor efficacy of Compound 1 (30 mg/kg, orally, once daily) alone or in combination with cetuximab in the CR1451 (KRAS^{G12C}) colorectal patient-derived tumor model. Single-agent Compound 1 treatment resulted in tumor stasis (64% tumor growth inhibition [TGI]), whereas single-agent cetuximab showed slowed growth inhibition (48% TGI). The combination of Compound 1 with cetuximab resulted in improved combination efficacy (83% TGI) relative to the single agents. All doses and combinations tested were tolerated based on minimal changes in body weight and overall animal condition.

**Table 8: Anti-Tumor Activity of Compound 1 and Cetuximab Dosed Alone or in Combination in CR1451 Colorectal Patient-Derived Xenograft Model in Nude Mice.**

| Treatment | Dose Levels (mg/kg), Schedule | TI | PR | CR | % TGI (estimated) | % TGI (lower CI) | % TGI (upper CI) |
|---|---|---|---|---|---|---|---|
| Vehicles | 0 (Vehicles) | 10/10 | 0 | 0 | 0 | 0 | 0 |
| Compound 1 | 30, PO, QD | 10/10 | 0 | 0 | 64 | 32 | 81 |
| Cetuximab | 20, IP, BIW | 10/10 | 0 | 0 | 48 | 4 | 73 |
| Compound 1 + Cetuximab | 30 + 20 | 10/10 | 1 | 0 | 83 | 66 | 93 |
| CI = confidence interval; CR = complete response; PR = partial response; | | | | | | | |
| QD = once daily; TI = tumor incidence. | | | | | | | |
| Vehicle = 0.5% (w/v) methylcellulose. | | | | | | | |

**Example 8:** *KRAS* is the most frequently mutated oncogene in up to 25% of cancers and is associated with resistance to select standard-of-care therapies and overall poor prognosis. Although selective inhibitors have been developed as anti-cancer therapy to target other nodes in the RAS/MAPK pathway, the KRAS oncoprotein was considered undruggable until the recent discovery of the switch II pocket (Ostrem, et al. Nature 2013;503:548-51). With this finding, covalent small molecule inhibitors aimed at targeting KRAS, and specifically the KRAS^{G12C} mutation, are being evaluated in early clinical development.

**Other KRAS^{G12C} inhibitors.** AMG 510 (sotorasib) is a small molecule that irreversibly inhibits KRAS^{G12C} by locking it in its inactive GDP-bound state. AMG-510 is currently being investigated in ongoing clinical studies. Patients in those studies received a median of 3 (range, 0 to 11) prior lines of anti-cancer therapies for metastatic disease before entering the study. Overall, treatment-related adverse events were reported in 56.6% of patients; 11.6% of patients experienced a treatment-related Grade 3 or 4 event, and 1.6% of patients experienced a treatment-related serious adverse event. Grade 3 events occurring in more than one patient included ALT increase, diarrhea, anemia, AST increase, and alkaline phosphatase increase. One patient experienced Grade 4 treatment-related ALT increase, and one patient discontinued AMG 510 due to Grade 3 treatment-related ALT and AST increase. While anti-tumor activity was reported, adverse events associated with AMG-510 exist. Patients had a confirmed objective response in 32.2% of patients with NSCLC and the median duration of response was 10.9 months (range, 1.1+ to 13.6) in patients. Median PFS was reported to be 6.3 months (range, 0.0+ to 14.9+) in patients with NSCLC (Hong et al. New Eng J Med 2020;383:1207-17).

MRTX849 is a mutant-selective small molecule KRAS^{G12C} inhibitor being evaluated in a clinical study of patients with advanced solid tumors with the KRAS^{G12C} mutation. Data from a total of 17 patients (including 10 patients with NSCLC and 4 patients with CRC), of which 12 patients had undergone at least one on-treatment tumor assessment (including 6 patients with NSCLC and 4 patients with CRC), were reported recently. Most patients had received 3 or more prior anti-cancer regimens before study entry (12 of 17 patients, 71%). The following treatment-related adverse events were reported in > 10% of patients: diarrhea, nausea, AST increased, vomiting, fatigue, ALT increased, creatinine increased, abdominal distension, abdominal pain, ALP increased, anemia, decreased appetite, dehydration, dry mouth, dysgeusia, dyspnea, QT prolonged, hypomagnesemia, and rash. Grade 3 events included fatigue, decreased appetite, and dyspnea (1 patient each). Anti-tumor activity with PR was achieved in 3 of 6 patients with NSCLC and 1 of 4 patients with CRC across all dose levels evaluated (Jänne et al. AACR-NCI-EORTC International Conference on Molecular Targets and Cancer Therapeutics October 2019).

**Compound 1.** The specificity of Compound 1 for KRAS^{G12C} , together with its mechanism of action, leads to potent and irreversible inhibition of KRAS^{G12C}, and is expected to enable a broad therapeutic index, maximizing anti-tumor activity while minimizing treatment-related toxicities. Specific therapies aimed at KRAS^{G12C}-positive cancer may provide more tolerable and effective treatment options for patients with advanced stage cancers that harbor KRAS^{G12C}.

In vitro and in vivo pharmacology studies demonstrate that Compound 1 is a highly potent and selective covalent inhibitor of KRAS^{G12C} , exhibiting over 20,000-fold selectivity in growth inhibition for KRAS^{G12C}-positive over KRAS^{G12C}-negative cancer cell lines. Mechanism of action studies with Compound 1 demonstrate that downstream MAPK pathway components such as phosphorylated (p)ERK and pS6, in addition to KRAS target genes such as DUSP6 and SPRY4, are inhibited and apoptosis induction is observed in KRAS^{G12C}-positive cancer cell lines. In addition, Compound 1 has potent single-agent activity and inhibits tumor growth in a number of nonclinical xenograft models of KRAS^{G12C}-positive lung tumors. These in vitro and in vivo pharmacology studies support the use of Compound 1 for the treatment of patients with locally advanced or metastatic KRAS^{G12C} -positive solid tumors.

The results of nonclinical toxicology studies completed to date provide a robust characterization of the toxicity profile of Compound 1 and support the administration of Compound 1 in patients with cancer. Comprehensive nonclinical toxicity studies were completed to evaluate the potential single and repeat dose oral toxicity, genetic toxicity, phototoxicity, and safety pharmacology of Compound 1. Because the KRAS^{G12C} mutation is not present in healthy animals, there are no pharmacologically relevant nonclinical species for KRAS^{G12C} inhibition.

Cetuximab is a recombinant, human/mouse chimeric monoclonal antibody that binds specifically to the extracellular domain of the human epidermal growth factor receptor (EGFR). Cetuximab is composed of the Fv regions of a murine anti-EGFR antibody with human IgG1 heavy and kappa light chain constant regions and has an approximate molecular weight of 152 kDa. Cetuximab is produced in mammalian (murine myeloma) cell culture. In one embodiment, cetuximab is marketed under the tradename ERBITUX^{®}.

Cetuximab is approved for the treatment of a number of different solid tumor types, including metastatic colorectal cancer and head and neck cancer. Erlotinib is approved for the treatment of non-small cell lung cancer (NSCLC), in particular NSCLC tumors having epidermal growth factor receptor (EGFR) exon 19 deletions or exon 21 (L858R) substitution mutations as detected by an FDA-approved test receiving first-line, maintenance, or second or greater line treatment after progression following at least one prior chemotherapy regimen. Erlotinib is also approved for first-line treatment of locally advanced, unresectable or metastatic pancreatic cancer, in combination with gemcitabine.

Early Phase I clinical data from the ongoing studies of AMG 510 and MRTX849 as single agents have shown that KRAS^{G12C} inhibitors are tolerable and have promising anti-tumor activity in patients with metastatic NSCLC and CRC (Janne et al. 2019; Hong et at. New Eng J Med 2020;383:1207-17). However, there still remains a great unmet need to improve upon the anti-tumor activity and durability reported in NSCLC and CRC with this class of inhibitors as a single agent while importantly retaining their tolerable safety profile.

**Rationale for Combination Therapy with an EGFR-inhibitor.** Without being bound by any particular theory, and based on the mechanistic understanding of the RTK-RAS-MAPK pathway, it has been hypothesized that inhibition upstream of KRAS^{G12C} with RTK inhibitors could potentially enhance KRAS^{G12C} inhibition. Nonclinical studies as described in Example 1 in cell lines support this strategy by showing that EGFR inhibition, with either small molecules or anti-EGFR antibodies that inhibit the activity of wild-type EGFR, synergistically enhanced KRAS^{G12C} inhibition (Lito et al. Science 2016;351:604-8; Canon et al. Nature 2019;575:217-23; Amodio et al. Cancer Disc 2020;10:1129-39; Hallin et al. Cancer Disc 2020;10:54-71). Possible mechanisms by which EGFR inhibition enhance the effect of KRAS^{G12C} inhibitors include reducing nucleotide exchange to favor the GDP-bound state of KRAS^{G12C} (Lito et al. 2016) and reducing the rebound enhancement of RTK signaling upon KRAS^{G12C} inhibition (Amodio et al. 2020).

Combination with Cetuximab. In *in vivo* mouse studies, treatment of mice with CRC PDXs using the combination of Compound 1 and cetuximab reduced tumor growth beyond that seen with Compound 1 alone. The nonclinical evidence (See FIGs. 3-8 and Examples 2-7) shows synergy between EGFR inhibition and KRAS^{G12C} inhibition in CRC. The starting dose of cetuximab in combination with Compound 1 will be an initial dose of 400 mg/m² as a 120-minute IV infusion on Day 1 followed by 250 mg/m² as a 60-minute IV infusion weekly, in 21-day cycles. Potential overlapping toxicities of the agents include gastrointestinal toxicities and elevated hepatic transaminases.

Combination with Erlotinib. In multiple KRAS^{G12C}-positive cell lines, Compound 1 in combination with erlotinib showed a synergistic effect on inhibition of cell growth along with corresponding reduction in pERK and pS6 greater than the effect seen with Compound 1 alone. In *in vivo* mouse studies, greater reduction in tumor growth of NSCLC xenografts was achieved with Compound 1 and erlotinib compared with Compound 1 alone. The nonclinical evidence (See FIG. 1 and FIG. 2) shows synergy between EGFR inhibition and KRAS^{G12C} inhibition in NSCLC. The starting dose of erlotinib in combination with Compound 1 will be 150 mg PO QD in 21-day cycles. Potential overlapping toxicities of the agents include gastrointestinal toxicities and elevated hepatic transaminases.

Biomarkers. This study will identify and/or evaluate biomarkers that are predictive of response to Compound 1 as a single agent or in combination with an EGFR-inhibitor (i.e., predictive biomarkers), early surrogates of activity, associated with progression to a more severe disease state (i.e., prognostic biomarkers), associated with acquired resistance to KRAS^{G12C} inhibitors (e.g., Compound 1), associated with susceptibility to developing adverse events or can lead to improved adverse event monitoring or investigation (i.e., safety biomarkers), can provide evidence of Compound 1 activity in combination with an EGFR-inhibitor (i.e., pharmacodynamic [PD] biomarkers), or can increase the knowledge and understanding of disease biology and drug safety. Corresponding biomarker endpoints include the relationship between exploratory biomarkers in blood, plasma, and tumor tissue and safety, PK, activity, or other biomarker endpoints.

Patients are screened for period of up to 28 days, followed by a treatment period, and a safety follow-up period during which patients will be followed for safety outcomes for a treatment-specific period after their final dose of study drug or until they receive another anti-cancer therapy, whichever occurs first.

In the absence of unacceptable toxicities and unequivocal disease progression as determined by the investigator, patients may continue treatment with Compound 1.

All patients will be closely monitored for adverse events throughout the study and for a treatment-specific period after the final dose of study treatment or until initiation of another anti-cancer therapy, whichever occurs first. Adverse events will be graded according to the NCI CTCAE v5.0.

The starting dose of Compound 1 will be 50 mg PO QD. Single-patient dose-escalation cohorts will be treated at escalating dose levels of Compound 1.

Patients include those with locally advanced, recurrent, or metastatic incurable KRas^{G12C}-positive tumors (e.g. NSCLC, CRC, or pancreatic cancer) who have disease progression or intolerance to at least one prior systemic therapy that may include single-agent or combination therapy. Patient having NSCLC, CRC, or pancreatic cancer will be screened for KRas^{G12C}-positivity.

**KRas^{G12C} Mutation Status from Tissue and Circulating Tumor DNA Assessments.** Approximately 12% of NSCLC, 4% of CRC, 2% of pancreatic cancers, and many other solid tumors (prevalence ≤ 4% in each) harbor the KRas^{G12C} mutation. Compound 1 is a potent and highly selective inhibitor that targets KRas^{G12C}, but not other mutations in KRAS, the wild-type form of KRAS, or other members of the RAS family. Therefore, only patients with tumors harboring the KRas^{G12C} mutation are eligible for administration of combination therapies described herein. KRAS mutation status may be determined using the FoundationOne^{®} CDx (F1CDx) assay, a U.S. Food and Drug Administration (FDA)-approved broad companion diagnostic (CDx) assay, FoundationOne^{®} Liquid CDx (F1L CDx) assay, as well as other FDA approved (FDA 2020) or well-validated laboratory developed tests performed in a Clinical Laboratory Improvement Amendments (CLIA)-validated or equivalently certified laboratory. Previous studies indicate that occurrence of the KRas^{G12C} mutation is an early event (Jamal-Hanjani et al. N Engl J Med 2017;376:2109-21), suggesting that analysis of archival tissue is a sufficient surrogate for selection of patients with KRas^{G12C}-positive tumors for Compound 1 treatment.

**Pharmacodynamic Pathway Modulation.** Compound 1 is a KRas^{G12C} inhibitor that suppresses downstream MAPK signaling by alkylation of KRas^{G12C}, thereby locking it in its inactive GDP-bound state. In nonclinical models, the level of KRas^{G12C} alkylation by Compound 1 and the extent of MAPK pathway suppression correlate with response to Compound 1 . Pre-treatment and on-treatment tumor tissue collection will enable an assessment of the correlation of MAPK pathway suppression and anti-tumor activity with Compound 1 treatment. The extent of MAPK pathway suppression can be assessed using RNA analysis of MAPK target genes (e.g., DUSP6, SPRY4) or immunohistochemistry (IHC) analysis of phosphorylated downstream markers (e.g., pERK, pS6). In addition, on-treatment tumor tissue biopsies may enable direct assessment of the level of KRas^{G12C} alkylation by Compound 1. The assessment of these PD biomarkers may inform future dose selection.

**Sequencing of Genes Related to Resistance to Compound 1.** DNA sequencing techniques, such as targeted next-generation sequencing (NGS) and whole exome sequencing, may offer a unique opportunity to identify biomarkers of response and/or resistance to Compound 1. Sequencing of cancer-related genes may result in the identification of de novo and acquired mechanisms of resistance to Compound 1.

**Protein, RNA, and DNA Analysis.** In addition to mutational activation of proteins, expression levels of RNA or alterations in DNA may also modulate the activity of signaling pathways. RNA profiling of tumors will allow intrinsic subtyping of patients enrolled in the study. Analysis of the potential association between subtypes and patient outcome may identify subpopulations of patients who are most likely to respond to Compound 1.

**Plasma Sample for Somatic Tumor Mutation Analysis and Other Biomarkers.** There is increasing evidence that cell-free DNA obtained from blood specimens of patients with cancer contains ctDNA, which is representative of the DNA and mutational status of cells in the tumor (Diehl et al. 2008; Maheswaran et al. 2008). Assays have been validated to detect cancer-related mutations (e.g., KRAS) from plasma. Results of these assays may be correlated with the mutational status determined from analysis of tumor specimens. The use of ctDNA to monitor response to treatment is an area of great interest, and could allow for an early, non-invasive, and quantifiable method for use in the clinical setting to identify candidates for specific therapies and monitoring of mutation status of the cancer over time (Wan et al. Nat Rev Cancer 2017;17:223-38). Analysis of ctDNA collected at various times during study treatment and after a patient progresses on Compound 1 may help to identify mechanisms of response and acquired resistance to study treatment.

**Blood Sample for Next-Generation Sequencing.** Next-generation sequencing (NGS) technologies can generate a large quantity of sequencing data. Tumor DNA can contain both reported and unreported chromosomal alterations because of the tumorigenesis process. To help control for sequencing calls in previously unreported genomic alterations, a predose blood sample will be taken to determine whether the alteration is somatic.

**Inclusion Criteria.** Patients must meet the following criteria for study entry:
- Age ≥ 18 years;
- Evaluable or measurable disease per RECIST v1.1;
- Eastern Cooperative Oncology Group (ECOG) performance status of 0 or 1;
- Life expectancy of ≥12 weeks;
- Adequate hematologic and organ function within 14 days prior to initiation of study treatment, defined by the following:
   ∘ Absolute neutrophil count ≥1200/µL;
   ∘ Hemoglobin ≥9 g/dL;
   ∘ Platelet count ≥100,000/µL;
   ∘ Total bilirubin ≤1.5 × ULN;
   ∘ Serum albumin ≥2.5 g/dL;
   ∘ AST and ALT ≤2.5 × ULN with the following exception:
      ▪ Patients with documented liver metastases may have AST and/or ALT ≤5.0 × ULN.
   ∘ Serum creatinine ≤1.5 × ULN or creatinine clearance ≥50 mL/min on the basis of the Cockcroft-Gault glomerular filtration rate estimation: $\left(140-age\right) \times \left(weight in kg\right) \times \left(0.85 if female\right)$ $72 \times \left(serum creatinine in mg/dL\right)$
- For women of childbearing potential: Agreement to remain abstinent (refrain from heterosexual intercourse) or use contraception, and agreement to refrain from donating eggs;
- For men who are not surgically sterile: Agreement to remain abstinent (refrain from heterosexual intercourse) or use contraception, and agreement to refrain from donating sperm;
- Confirmation of biomarker eligibility: Valid results from either central testing of blood or local testing of blood or tumor tissue documenting the presence of the KRas^{G12C} mutation (e.g. validated polymerase chain reaction (PCR)-based or NGS assay performed at a CLIA or equivalently certified laboratory).

### Additional inclusion criteria

- Histologically documented, locally advanced, recurrent, or metastatic incurable adenocarcinoma of the colon or rectum, without a known concomitant second oncogenic driver (e.g., BRAF V600E mutation, ERBB2 amplification) as determined by the FMI NGS assay or by a Sponsor-approved validated PCR-based or NGS assay performed at a local CLIA-certified or equivalently-certified laboratory.
   o Patients with appendiceal tumors are excluded
   o Patients must have experienced disease progression or intolerance to at least one prior chemotherapy regimen (e.g., FOLFOX, FOLFIRI, FOLFOXIRI ± bevacizumab)
- Histologically documented, locally advanced, recurrent, or metastatic incurable NSCLC, without a known concomitant second oncogenic driver (e.g., sensitizing EGFR mutations, ALK rearrangement, ROS1 rearrangement, BRAF V600E mutation, NTRK fusions, RET fusions) as determined by the FMI NGS assay or by a Sponsor-approved validated PCR-based or NGS assay performed at a local CLIA-certified or equivalently-certified laboratory.
   o Disease progression or intolerance to at least 1 prior systemic therapy. This may include single-agent or combination therapy with an investigational or approved PD-L1/PD-1 inhibitor.
- Patients may have received prior treatment with a KRas^{G12C} specific inhibitor.

**General Exclusion Criteria.** Patients who meet any of the following criteria will be excluded:
- Inability or unwillingness to swallow pills;
- Inability to comply with study and follow-up procedures;
- Malabsorption syndrome or other condition that interferes with enteral absorption;
- Known and untreated, or active central nervous system (CNS) metastases;
- Patients with a history of treated CNS metastases provided they meet all of the following criteria:
   ∘ Measurable or evaluable disease outside the CNS;
   ∘ No history of intracranial hemorrhage or spinal cord hemorrhage;
   ∘ No ongoing requirement for corticosteroids as therapy for CNS metastases, with corticosteroids discontinued for ≥ 2 weeks prior to administration of an agent described herein and no ongoing symptoms attributed to CNS metastases;
   o No stereotactic radiation within 7 days or whole-brain radiation within 14 days prior to Day 1 of Cycle 1;
   ∘ No evidence of interim progression between the completion of CNS-directed therapy and the screening radiographic study;
- Leptomeningeal disease or carcinomatous meningitis;
- **Uncontrolled** pleural effusion, pericardial effusion, or ascites requiring recurrent drainage procedures biweekly or more frequently;
   o Indwelling pleural or abdominal catheters may be allowed, provided the patient has adequately recovered from the procedure, is hemodynamically stable and symptomatically improved;
- Any active infection that could impact patient safety, or serious infection requiring **IV** antibiotics within 7 days prior to Day 1 of Cycle 1;
- Clinically significant history of liver disease, including viral or other hepatitis, current alcohol abuse, or cirrhosis;
- Known **HIV** infection;
- Uncontrolled hypercalcemia (> 1.5 mmol/L ionized calcium or calcium > 12 mg/dL or corrected serum calcium ≥ ULN) or symptomatic hypercalcemia requiring continued use of bisphosphonate therapy or denosumab;
- Significant traumatic injury or major surgical procedure within 4 weeks prior to Day 1 of Cycle 1;
- Patients with chronic diarrhea, short bowel syndrome or significant upper gastrointestinal surgery including gastric resection, a history of inflammatory bowel disease (e.g., Crohn's disease or ulcerative colitis) or any active bowel inflammation (including diverticulitis);
- Treatment with chemotherapy, immunotherapy, or biologic therapy as anti-cancer therapy within 3 weeks prior to administration of an agent described herein, or endocrine therapy within 2 weeks prior administration of an agent described herein, except for the following:
   ∘ Hormonal therapy with gonadotropin-releasing hormone (GnRH) agonists or antagonists for endocrine sensitive cancers (e.g., prostate, endometrial, hormone receptor-positive breast cancer);
   o Kinase inhibitors, approved by regulatory authorities, may be used up to 2 weeks prior to initiation of study treatment,;
   o Treatment with an investigational agent within 3 weeks or five half-lives prior to administration of an agent described herein, whichever is shorter.
- Radiation therapy (other than palliative radiation to bony metastases and radiation to CNS metastases) as cancer therapy within 4 weeks prior to administration of an agent described herein;
- Palliative radiation to bony metastases within 2 weeks prior to administration of Compound 1;
- Adverse events from prior anti-cancer therapy that have not resolved;
- History of other malignancy within 5 years prior to screening;
- History of or active clinically significant cardiovascular dysfunction, including:
   ∘ History of stroke or transient ischemic attack within 6 months prior to administration of an agent described herein;
   o History of myocardial infarction within 6 months prior to administration of an agent described herein;
   o New York Heart Association Class III or IV cardiac disease or congestive heart failure requiring medication
   o Uncontrolled arrhythmias, history of or active ventricular arrhythmia requiring medication;
   o Coronary heart disease that is symptomatic or unstable angina;
   o Congenital long QT syndrome or QT interval corrected through use of Fridericia's formula (QTcF) > 470 ms;
   o Current treatment with medications known to prolong the QT interval;
- Pregnant or breastfeeding, or intending to become pregnant during the study or within 6 months after the final dose of Compound 1; or
- History of idiopathic pulmonary fibrosis, organizing pneumonia (e.g., bronchiolitis obliterans), drug-induced pneumonitis, or idiopathic pneumonitis, or evidence of active pneumonitis on screening chest computed tomography (CT) scan;

Study Treatment Formulation, Packaging, and Handling.

Compound 1 will be supplied as an active pharmaceutical ingredient (API) powder-in-capsule (PIC) formulation in three strengths: 5 mg, 25 mg, and 100 mg (free base equivalent). Additionally, a film-coated tablet formulation in a dose strength of 100 mg (free base equivalent) will also be supplied for clinical use. Compound 1 drug products should be stored at or below 86°F (30°C) and protected from moisture.

For Compound 1 doses to be administered at home, a sufficient number of capsules or tablets should be dispensed to the patient to last until the next visit or through one cycle. Patients will self-administer Compound 1 as provided herein, except when patients visit a clinic. Patients should take Compound 1 at approximately the same time each day unless otherwise instructed. Patients will be instructed as to the number and strength of capsules or tablets to take, according to their assigned dose level and schedule.

Unless otherwise instructed, Compound 1 should be taken on an empty stomach, i.e., food should be avoided at least 2 hours before as well as 1 hour after the dose is administered. There are no restrictions on water intake. Importantly, Compound 1 capsules or tablets will be swallowed whole (not chewed) with a minimum of 240 mL (8 fluid ounces) of water. If a patient misses any dose of Compound 1 or vomits up a capsule or tablet, the patient should be instructed to skip that dose and resume dosing with the next scheduled dose. Missed doses will not be made up.

Cetuximab will be supplied in commercially available formulations. Cetuximab will be administered at an initial dose of 400 mg/m² as a 120-minute IV infusion on Day 1 followed by 250 mg/m² as a 60-minute IV infusion weekly, in 21-day cycles. The maximum infusion rate must not exceed 10 mg/min. Cetuximab should be administered following administration of Compound 1.

Administration of cetuximab will be performed in a monitored setting where there is immediate access to trained personnel and adequate equipment and medicine to manage potentially serious reactions. Prior to the first infusion, participants must receive premedication with an antihistamine and a corticosteroid. This premedication is recommended prior to all subsequent infusions. Close monitoring is required during the infusion and for at least 1 hour after the end of the infusion

Erlotinib will be supplied as tablets in 25mg, 100mg, and 150mg strengths. Erlotinib will be administered PO QD starting at 150 mg in 21-day cycles, at the same time as Compound 1, with sips of water in between. All doses of erlotinib should be taken on an empty stomach (i.e., food should be avoided at least 2 hours before as well as 1 hour after the dose is administered).

In the event erlotinib or cetuximab administration is held due to an adverse event in a given cycle, the next dosing cycle should not begin until administration of erlotinib or cetuximab can be resumed. As such, the current cycle may be extended past 21 days, and the patient may continue to receive Compound 1. Day 1 of the next cycle should correspond to the timepoint at which administration of erlotinib or cetuximab is resumed.

**Concomitant Therapy.** Concomitant therapy consists of any medication (e.g. prescription drugs, over-the-counter drugs, vaccines, herbal or homeopathic remedies, nutritional supplements) used by a patient in addition to an agent described herein from 7 days prior to the first administration of at least one agent described herein to the last administration of at least one agent described herein.

**Permitted Therapy.** Patients may take (a) anti-seizure medications or warfarin; (b) oral contraceptives or other allowed maintenance therapy as specified in the eligibility criteria; (c) anti-emetics and anti-diarrheal medications should not be administered prophylactically before initial treatment with study drug; (d) pain medications; (e) bisphosphonate and denosumab therapy for bone metastases or osteopenia or osteoporosis; or multivitamins, calcium, and vitamins C, D, and E supplements are allowed.

**Precautionary Therapy.** Medications Given with Precaution due to Effects Related to CYP Enzymes and Compound 1 include, for example, (1) Strong/moderate CYP3A4 inhibitors, including, but not limited to, the following: atazanavir, ritonavir, indinavir, nelfinavir, saquinavir, clarithromycin, telithromycin, erythromycin, troleandomycin, fluconazole, itraconazole, ketoconazole, voriconazole, posaconazole, aprepitant, conivaptan, fluvoxamine, diltiazem, nefazodone, mibefradil, verapamil, and grapefruit juice or grapefruit supplements; (2) Strong/moderate CYP3A4 inducers, including, but not limited to, the following: rifampin, carbamazepine, phenytoin, oxcarbazepine, phenobarbital, efavirenz, nevirapine, etravirine, modafinil, hyperforin (St. John's Wort), and cyproterone. The use of full-dose oral or parenteral anticoagulants for therapeutic purpose as long as the INR and/or aPTT is within therapeutic limits (according to institution standards) within 14 days prior to administration of any agent described herein and the patient has been on a stable dose of anticoagulants for ≥ 1 week prior to initiation of study treatment. The lists of medications are not intended to be comprehensive.

Coumarins (Coumadin^{®}, warfarin) are strongly discouraged during erlotinib therapy. If the patient requires anticoagulation therapy, then the use of low molecular-weight heparin instead of coumarins is recommended, where clinically feasible. If there is no clinically feasible alternative to coumarins, frequent monitoring of INR and prothrombin time must be performed.

Drugs reducing gastric acid production, such as proton-pump inhibitors or H₂- receptor antagonists, have been shown to decrease erlotinib exposure. Therefore, co-administration of these drugs with erlotinib should be avoided. If the use of antacids is considered necessary during treatment with erlotinib, they should be taken at least 4 hours before or 2 hours after the daily dose of erlotinib.

Chronic use of anti-angiogenic agents and nonsteroidal anti-inflammatory drugs (NSAIDs) are not permitted for patients receiving erlotinib, as they may increase the risk of GI perforation. The acute use of NSAIDs is allowed for managing fever or during periods when erlotinib is held.

**Prohibited Therapy.** Use of the following concomitant therapies is prohibited during and for at least 7 days prior to the first administration of an agent described herein:
- Investigational therapy within 3 weeks or five half-lives prior to the first administration of an agent described herein, whichever is shorter;
- Concomitant therapy intended for the treatment of cancer whether approved by the FDA or experimental, including chemotherapy, radiotherapy, immunotherapy, biologic therapy, herbal therapy, or hormonal therapy except for the following:
   ∘ Hormonal therapy with gonadotropin-releasing hormone (GnRH) agonists or antagonists for endocrine sensitive cancers (e.g. prostate, endometrial, hormone receptor-positive breast cancer);
   o Hormone replacement therapy or oral contraception.
- Radiotherapy for unequivocal progressive disease with the exception of new brain metastases in the setting of systemic response: patients who have demonstrated control of their systemic disease (defined as having received clinical benefit [i.e., a PR, CR, or SD for ≥3 months]), but who have developed brain metastases that are treatable with radiation, will be allowed to continue to receive therapy with Compound 1 during the study until they either experience systemic progression of their disease and/or further progression in the brain (based on investigator assessments);
- Quinidine or other anti-arrhythmic agents;
- Initiation or increased dose of hematopoietic colony-stimulating factors (CSFs; e.g., granulocyte CSF; filgrastim, granulocyte/macrophage CSF; sargramostim, pegfilgrastim, erythropoietin, darbepoetin, and thrombopoietin) from 7 days before Cycle 1, Day 1

**Risks Associated with Compound 1.** Administration of Compound 1 has been associated diarrhea, nausea, vomiting, oral mucosal irritation, minimal to mild transaminase elevation, and phototoxicity.

**Risks Associated with cetuximab.** Undesirable effects of cetuximab include skin reactions which occur in more than 80% of patients, hypomagnesaemia which occurs in more than 10% of patients, and IRRs which occur with mild to moderate symptoms in more than 10% of patients and with severe symptoms in more than 1% of patients. The risk of serious infusion reactions with cetuximab administration may be increased in patients who have had a tick bite or have a red meat allergy.

**Risks Associated with erlotinib.** Erlotinib has been associated with the following risks: cutaneous toxicity, interstitial lung disease (ILD), liver injury, gastrointestinal (GI) fluid loss, GI perforation, and ocular toxicity. Current smokers should be advised to stop smoking as plasma concentrations of erlotinib in smokers are reduced compared to non-smokers. The degree of reduction is likely to be clinically significant. Potent inducers of CYP3A4 may reduce the efficacy of erlotinib whereas potent inhibitors of CYP3A4 may lead to increased toxicity. Erlotinib is a potent inhibitor of CYP1A1, and a moderate inhibitor of CYP3A4 and CYP2C8, as well as a strong inhibitor of glucuronidation by UGT1A1 in vitro. Please refer to the Erlotinib SmPC for complete drug-drug interaction information.

**Treatment Interruption.** If Compound 1 is held for > 21 days from the previous study treatment due to toxicity, the study treatment should not be re-initiated. Compound 1 may be suspended for up to 21 days for unanticipated intercurrent medical events that are not associated with study treatment toxicity or disease progression.

**Adverse Events.** An adverse event as defined herein refers to any untoward medical occurrence in a clinical investigation subject administered an agent described herein in the combination therapies described herein, regardless of causal attribution. The terms "severe" and "serious" are not synonymous. Severity refers to the intensity of an adverse event (e.g., rated as mild, moderate, or severe, or according to NCI CTCAE); the event itself may be of relatively minor medical significance (such as severe headache without any further findings).

Adverse events to be monitored include nausea, vomiting, diarrhea, stomatitis, mucositis, hepatitis or elevation in ALT or AST, elevated bilirubin or clinical jaundice, systemic lupus erythematosus, nephritis, Events suggestive of hypersensitivity, infusion-mediated reactions, CRS, influenza-like illness, and systemic inflammatory response syndrome , atrial fibrillation, myocarditis, pericarditis, Vasculitis, Myositis , uveitis, retinitis, optic neuritis, autoimmune hemolytic anemia, Stevens-Johnson syndrome, dermatitis bullous, and toxic epidermal necrolysis.

Throughout this specification and the claims, the words "comprise," "comprises," and "comprising" are used in a non-exclusive sense, except where the context requires otherwise. It is understood that embodiments described herein include "consisting of" and/or "consisting essentially of' embodiments.

Where a range of values is provided, it is understood that each intervening value, to the tenth of the unit of the lower limit, unless the context clearly dictates otherwise, between the upper and lower limit of the range and any other stated or intervening value in that stated range, is encompassed herein. The upper and lower limits of these small ranges which can independently be included in the smaller rangers is also encompassed herein, subject to any specifically excluded limit in the stated range. Where the stated range includes one or both of the limits, ranges excluding either or both of those included limits are also included herein.

Many modifications and other embodiments of the inventions set forth herein will come to mind to one skilled in the art to which these inventions pertain having the benefit of the teachings presented in the foregoing descriptions and the associated drawings.

Although specific terms are employed herein, they are used in a generic and descriptive sense only and not for purposes of limitation.

## Claims

1. A combination comprising:
(a) Compound 1 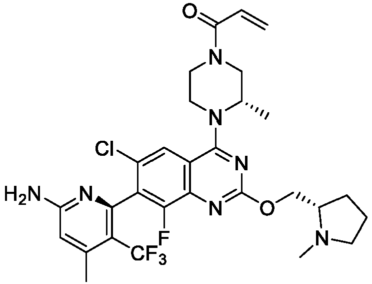 or a pharmaceutically acceptable salt thereof; and
(b) an EGFR-inhibitor selected from the group consisting of erlotinib, gefitinib, osimertinib, dacomitinib, and afatinib,
for use in the treatment of lung cancer mediated by a KRas^{G12C} mutation in a patient having such a lung cancer,
wherein Compound 1 is administered QD on days 1-21 of a first 21-day cycle.

2. The combination for use according to claim 1, wherein the lung cancer is NSCLC.

3. The combination for use according to claim 1, wherein the lung cancer is adenocarcinoma, squamous-cell lung carcinoma or large-cell lung carcinoma.

4. The combination for use according to any one of claims 1-3, wherein the EGFR-inhibitor is erlotinib.

5. The combination for use according to any one of claims 1-4, wherein the EGFR-inhibitor is erlotinib administered QD on days 1-21 of the first 21-day cycle.

6. The combination for use according to any one of claims 1-5, wherein the EGFR-inhibitor is erlotinib administered at an amount of about 150mg QD.

7. A combination comprising:
(a) Compound 1 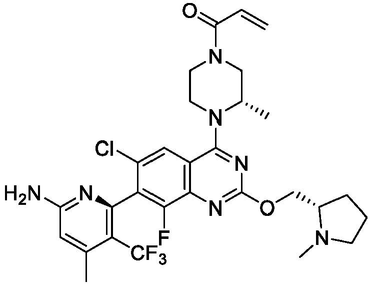 or a pharmaceutically acceptable salt thereof; and
(b) an anti-EGFR antibody comprising panitumumab or cetuximab
for use in the treatment of colorectal cancer (CRC) mediated by a **KRas^{G12C}** mutation in a patient having CRC,
wherein Compound 1 is administered QD on days 1-21 of a first 21-day cycle.

8. The combination for use according to claim 7, wherein the EGFR-inhibitor is cetuximab.

9. The combination for use according to any one of claims 7-8, wherein the EGFR-inhibitor is cetuximab administered at an amount of about 400 mg/m2 on day 1 of the 21-day cycle and at an amount of about 250 mg/m2 Q1W thereafter.

10. The combination for use according to any one of claims 1-9, wherein Compound 1 is an adipate salt thereof.

11. The combination for use according to any one of claims 1-10, wherein Compound 1 or a pharmaceutically acceptable salt thereof is administered orally as a tablet or capsule.

12. The combination for use according to any one of claims 1-11, wherein Compound 1 or a pharmaceutically acceptable salt thereof is administered at an amount of about 50mg-500mg.

13. The combination for use according to any one of claims 1-12 wherein Compound 1 or a pharmaceutically acceptable salt thereof is administered at an amount of about 100mg, 200mg, 300mg, 400mg, 500mg, 600mg, 700mg, or 800mg.

14. The combination for use according to any one of claims 1-13, wherein Compound 1 or a pharmaceutically acceptable salt thereof is administered at an amount of about 200mg or 400mg.

15. The combination for use according to any one of claims 1-14, wherein Compound 1 or a pharmaceutically acceptable salt thereof is administered at an amount of about 400mg.

16. The combination for use according to claim 4 or claim 8, wherein the patient has received treatment with at least one prior therapy or combination therapy.

17. The combination for use according to any one of claims 1-16, wherein the patient has been previously treated with a **KRas^{G12C}** specific inhibitor.

18. The combination for use according to any one of claims 7-18, wherein the patient is further administered chemotherapy comprising FOLFOX (leucovorin, fluorouracil, and oxaliplatin) or FOLFIRI (leucovorin, fluorouracil, and irinotecan).

## Patentansprüche

1. Kombination, umfassend:
(a) Verbindung 1 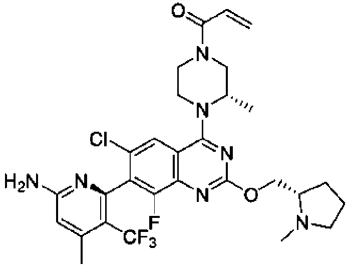 oder ein pharmazeutisch verträgliches Salz davon; und
(b) einen EGFR-Hemmer, ausgewählt aus der Gruppe, bestehend aus Erlotinib, Gefitinib, Osimertinib, Dacomitinib und Afatinib,
zur Verwendung bei der Behandlung von Lungenkrebs, der durch eine KRas^{G12C}-Mutation vermittelt wird, bei einem Patienten, der einen solchen Lungenkrebs hat, wobei Verbindung 1 QD an den Tagen 1-21 eines ersten 21-Tage-Zyklus verabreicht wird.

2. Kombination zur Verwendung nach Anspruch 1, wobei der Lungenkrebs NSCLC ist.

3. Kombination zur Verwendung nach Anspruch 1, wobei der Lungenkrebs Adenokarzinom, Plattenepithel-Lungenkarzinom oder großzelliges Lungenkarzinom ist.

4. Kombination zur Verwendung nach einem der Ansprüche 1-3, wobei der EGFR-Hemmer Erlotinib ist.

5. Kombination zur Verwendung nach einem der Ansprüche 1-4, wobei der EGFR-Hemmer Erlotinib ist, das QD an den Tagen 1-21 des ersten 21-Tage-Zyklus verabreicht wird.

6. Kombination zur Verwendung nach einem der Ansprüche 1-5, wobei der EGFR-Hemmer Erlotinib ist, der in einer Menge von etwa 150 mg QD verabreicht wird.

7. Kombination, umfassend:
(a) Verbindung 1 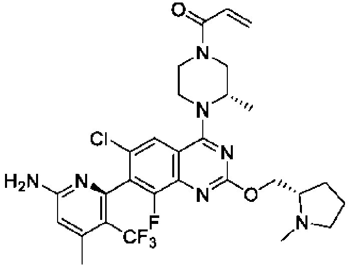 oder ein pharmazeutisch verträgliches Salz davon; und
(b) einen Anti-EGFR-Antikörper, umfassend Panitumumab oder Cetuximab, zur Verwendung bei der Behandlung von Kolorektalkrebs (CRC), der durch eine KRas^{G12C}-Mutation vermittelt wird, bei einem Patienten, der CRC hat,
wobei Verbindung 1 QD an den Tagen 1-21 eines ersten 21-Tage-Zyklus verabreicht wird.

8. Kombination zur Verwendung nach Anspruch 7, wobei der EGFR-Hemmer Cetuximab ist.

9. Kombination zur Verwendung nach einem der Ansprüche 7-8, wobei der EGFR-Hemmer Cetuximab ist, das an Tag 1 des 21-Tage-Zyklus in einer Menge von etwa 400 mg/m² und danach in einer Menge von etwa 250 mg/m² Q1W verabreicht wird.

10. Kombination zur Verwendung nach einem der Ansprüche 1-9, wobei Verbindung 1 ein Adipatsalz davon ist.

11. Kombination zur Verwendung nach einem der Ansprüche 1-10, wobei Verbindung 1 oder ein pharmazeutisch verträgliches Salz davon oral als Tablette oder Kapsel verabreicht wird.

12. Kombination zur Verwendung nach einem der Ansprüche 1-11, wobei Verbindung 1 oder ein pharmazeutisch verträgliches Salz davon in einer Menge von etwa 50 mg-500 mg verabreicht wird.

13. Kombination zur Verwendung nach einem der Ansprüche 1-12, wobei Verbindung 1 oder ein pharmazeutisch verträgliches Salz davon in einer Menge von etwa 100 mg, 200 mg, 300 mg, 400 mg, 500 mg, 600 mg, 700 mg oder 800 mg verabreicht wird.

14. Kombination zur Verwendung nach einem der Ansprüche 1-13, wobei Verbindung 1 oder ein pharmazeutisch verträgliches Salz davon in einer Menge von etwa 200 mg oder 400 mg verabreicht wird.

15. Kombination zur Verwendung nach einem der Ansprüche 1-14, wobei Verbindung 1 oder ein pharmazeutisch verträgliches Salz davon in einer Menge von etwa 400 mg verabreicht wird.

16. Kombination zur Verwendung nach Anspruch 4 oder Anspruch 8, wobei der Patient eine Behandlung mit mindestens einer vorherigen Therapie oder Kombinationstherapie erhalten hat.

17. Kombination zur Verwendung nach einem der Ansprüche 1-16, wobei der Patient zuvor mit einem KRas^{G12C}-spezifischen Hemmer behandelt wurde.

18. Kombination zur Verwendung nach einem der Ansprüche 7-18, wobei dem Patienten ferner eine Chemotherapie verabreicht wird, die FOLFOX (Leucovorin, Fluoruracil und Oxaliplatin) oder FOLFIRI (Leucovorin, Fluoruracil und Irinotecan) umfasst.

## Revendications

1. Association comprenant :
(a) le composé 1 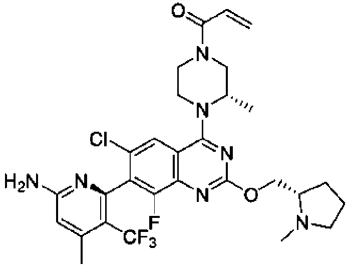 ou un sel pharmaceutiquement acceptable de celui-ci ; et
(b) un inhibiteur d'EGFR choisi dans le groupe constitué par l'erlotinib, le géfitinib, l'osimertinib, le dacomitinib et l'afatinib,
pour une utilisation dans le traitement d'un cancer du poumon médié par une mutation KRas^{G12C} chez un patient ayant un tel cancer du poumon,
dans laquelle le composé 1 est administré une fois par jour aux jours 1 à 21 d'un premier cycle de 21 jours.

2. Association pour une utilisation selon la revendication 1, dans laquelle le cancer du poumon est un CPNPC.

3. Association pour une utilisation selon la revendication 1, dans laquelle le cancer du poumon est un adénocarcinome, un carcinome pulmonaire à cellules squameuses ou un carcinome pulmonaire à grandes cellules.

4. Association pour une utilisation selon l'une quelconque des revendications 1 à 3, dans laquelle l'inhibiteur d'EGFR est l'erlotinib.

5. Association pour une utilisation selon l'une quelconque des revendications 1 à 4, dans laquelle l'inhibiteur d'EGFR est l'erlotinib administré une fois par jour aux jours 1 à 21 du premier cycle de 21 jours.

6. Association pour une utilisation selon l'une quelconque des revendications 1 à 5, dans laquelle l'inhibiteur d'EGFR est l'erlotinib administré en une quantité d'environ 150 mg une fois par jour.

7. Association comprenant :
(a) le composé 1 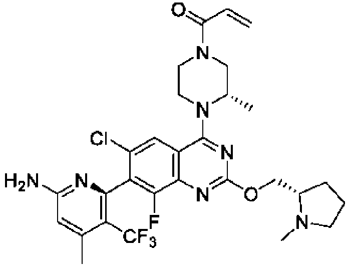 ou un sel pharmaceutiquement acceptable de celui-ci ; et
(b) un anticorps anti-EGFR comprenant le panitumumab ou le cétuximab pour une utilisation dans le traitement d'un cancer colorectal (CCR) médié par une mutation KRas^{G12C} chez un patient ayant un CCR,
dans laquelle le composé 1 est administré une fois par jour aux jours 1 à 21 d'un premier cycle de 21 jours.

8. Association pour une utilisation selon la revendication 7, dans laquelle l'inhibiteur d'EGFR est le cétuximab.

9. Association pour une utilisation selon l'une quelconque des revendications 7 à 8, dans laquelle l'inhibiteur d'EGFR est le cétuximab administré en une quantité d'environ 400 mg/m² au jour 1 du cycle de 21 jours et en une quantité d'environ 250 mg/m² une fois par semaine ensuite.

10. Association pour une utilisation selon l'une quelconque des revendications 1 à 9, dans laquelle le composé 1 est un sel d'adipate de celui-ci.

11. Association pour une utilisation selon l'une quelconque des revendications 1 à 10, dans laquelle le composé 1 ou un sel pharmaceutiquement acceptable de celui-ci est administré par voie orale sous forme de comprimé ou de gélule.

12. Association pour une utilisation selon l'une quelconque des revendications 1 à 11, dans laquelle le composé 1 ou un sel pharmaceutiquement acceptable de celui-ci est administré en une quantité d'environ 50 mg à 500 mg.

13. Association pour une utilisation selon l'une quelconque des revendications 1 à 12 dans laquelle le composé 1 ou un sel pharmaceutiquement acceptable de celui-ci est administré en une quantité d'environ 100 mg, 200 mg, 300 mg, 400 mg, 500 mg, 600 mg, 700 mg ou 800 mg.

14. Association pour une utilisation selon l'une quelconque des revendications 1 à 13, dans laquelle le composé 1 ou un sel pharmaceutiquement acceptable de celui-ci est administré en une quantité d'environ 200 mg ou 400 mg.

15. Association pour une utilisation selon l'une quelconque des revendications 1 à 14, dans laquelle le composé 1 ou un sel pharmaceutiquement acceptable de celui-ci est administré en une quantité d'environ 400 mg.

16. Association pour une utilisation selon la revendication 4 ou la revendication 8, dans laquelle le patient a reçu un traitement avec au moins une thérapie ou une association thérapeutique antérieure.

17. Association pour une utilisation selon l'une quelconque des revendications 1 à 16, dans laquelle le patient a été précédemment traité par un inhibiteur spécifique de KRas^{G12C}.

18. Association pour une utilisation selon l'une quelconque des revendications 7 à 18, dans laquelle le patient se voit en outre administrer une chimiothérapie comprenant FOLFOX (leucovorine, fluorouracile et oxaliplatine) ou FOLFIRI (leucovorine, fluorouracile et irinotécan).
